# EUROPEAN PATENT APPLICATION

(11) **EP 4 644 897 A1**
(43) Date of publication of application: **05.11.2025**
(21) Application number: 22970165.1
(22) Date of filing: 28.12.2022
(51) Int. Cl.: G01N 33/18, G01N 21/73, G01N 27/62, G01N 31/00

(54) **ANALYSIS METHOD FOR SAMPLE CONTAINING MERCURY**

(71) Applicant: BL TEC K.K., Osaka-shi, Osaka, 550-0002 (JP)
(72) Inventor: KUMAZAWA, Yorihiro, Tokyo 103-0011 (JP); KANNO, Ryosei, Tokyo 103-0011 (JP); TAKAHASHI, Nodoka, Tokyo 103-0011 (JP)
(74) Representative: Steffens, Adrian
(86) International application number: PCT/JP2022/048593
(87) International publication number: WO 2024/142378

(57) **Abstract**

For the purpose of providing an analysis method that makes it possible to use, for example, ICP-MS to analyze, with high accuracy, a sample containing mercury, a method for analyzing a sample containing mercury includes: a pretreatment step of adding an oxidizer to a sample containing mercury to prepare a sample solution so as to achieve an oxidation state in which an oxidation-reduction potential is not less than +0.80 V; and an analysis step of subjecting the sample solution which has been prepared in the pretreatment step to a quantitative analysis or a qualitative analysis while maintaining the oxidation state.

## Description

### Technical Field

The present invention relates to a method for analyzing a sample containing mercury.

### Background Art

As mercury was confirmed to be highly toxic, emissions and releases of mercury have been regulated in recent years. The Minamata Convention, which entered into force in 2017, prescribes the prohibition of mining of mercury and the regulations of the import and export of mercury and the like, with the aim of protecting the human health and the environment from the anthropogenic emissions and releases of mercury and mercury compounds. The EU also strictly regulates the use, the introduction, etc. of mercury under the RoSH Directive.

Mercury that is in a state of Hg²⁺ is stabilized by binding to, for example, Cl⁻ without vaporizing. In contrast, in a case where a reducing substance is present, mercury is reduced from Hg²⁺ to Hg, vaporizes during heating, and is lost by volatilization. Thus, in analyzing a sample containing mercury, it is impossible to obtain a good mercury recovery rate.

Therefore, in a reduction vaporization atomic absorption method, which is a typical mercury analysis method, as a pretreatment, a solution obtained by adding potassium permanganate to a sample containing mercury is heated so as to be brought into an oxidation state, so that the mercury is in a state of Hg²⁺ and is prevented from being lost by vaporization. The above method is designated as an official method in environmental water, waste water, drinking water, and the like (see Non-Patent Literatures 1 and 2).

### Citation List

### [Non-patent Literature]

[Non-patent Literature 1]
   The Ministry of Health, Labour and Welfare Notification No. 261, 2003, Appended Table 7
[Non-patent Literature 2]
   The Environment Agency Notification No. 59, 1971, Appended Table 2

### Summary of Invention

### Technical Problem

However, in a case where mercury is measured by the reduction vaporization atomic absorption method or the like, as a pretreatment, it is necessary to add sulfuric acid, nitric acid, and potassium permanganate to a sample. In a case where mercury is measured by an inductively coupled plasma mass spectrometry (ICP-MS) that makes it possible to simultaneously analyze, for example, sample solutions obtained by thus carrying out the pretreatment, e.g., almost all metal elements, sulfuric acid is added in a large amount. This causes such problems that sulfuric acid viscosity causes a problem in spraying from a nebulizer, and that there is mass interference with other substances which is caused by sulfur, and the like.

Further, in a case where the foregoing pretreatment is carried out with respect to a sample containing mercury and measurement is carried out by ICP-MS, there is room for improvement in terms of accuracy with which the sample containing mercury is detected.

An aspect of the present invention has been developed in view of the above problems and has an object to provide an analysis method that makes it possible to use, for example, ICP-MS to analyze, with high accuracy, a sample containing mercury.

### Solution to Problem

In order to attain the object, a method for analyzing a sample containing mercury in accordance with an aspect of the present invention includes: a pretreatment step of adding an oxidizer to a sample containing mercury to prepare a sample solution so as to achieve an oxidation state in which an oxidation-reduction potential is not less than +0.80 V; and an analysis step of subjecting the sample solution which has been prepared in the pretreatment step to a quantitative analysis or a qualitative analysis while maintaining the oxidation state.

In order to attain the object, a method for analyzing a sample containing mercury in accordance with an aspect of the present invention is carried out by a flow analysis method including: a sample introduction step of introducing, into a tube, a sample containing mercury; and an analysis step of carrying out a quantitative analysis or a qualitative analysis with respect to the sample, the method including: a pretreatment step of, prior to the sample introduction step, adding an oxidizer to the sample containing mercury to prepare a sample solution and/or a reagent addition step of adding the oxidizer to the sample so as to achieve an oxidation-reduction potential of not less than +0.80 V, the sample being transferred inside the tube; a heating step of carrying out a heat treatment with respect to the sample to which the oxidizer has been added; a cooling step of cooling the sample which has been subjected to the heat treatment and which is transferred inside the tube; and a gas-liquid separation step of removing gas which is present in the tube after cooling, a quantitative analysis or a qualitative analysis being carried out with respect to the sample from which the gas has been removed in the gas-liquid separation step.

### Advantageous Effects of Invention

An aspect of the present invention makes it possible to use, for example, ICP-MS to analyze, with high accuracy, a sample containing mercury.

### Brief Description of Drawings

- Fig. 1: is a drawing schematically illustrating a configuration of Configuration Example 1 of a flow analyzer which is used in an analysis method in accordance with Embodiment 2.
- Fig. 2: is a drawing schematically illustrating a configuration of Configuration Example 2 of the flow analyzer which is used in the analysis method in accordance with Embodiment 2.
- Fig. 3: is a drawing illustrating a part of the configuration of the flow analyzer of Configuration Example 1.
- Fig. 4: is a drawing illustrating a configuration of a marker detection section in the flow analyzer of Configuration Example 1.
- Fig. 5: is a drawing schematically illustrating how a plurality of segments which are separated by gas bubbles are produced inside a tube by a gas bubble segmentation section in the flow analyzer of Configuration Example 1.
- Fig. 6: is a drawing schematically illustrating gas bubbles inside a tube after a heat treatment in the flow analyzer of Configuration Example 1.
- Fig. 7: is a drawing schematically illustrating how gas which is present in a tube is removed by a gas-liquid separation section in the flow analyzer of Configuration Example 1.
- Fig. 8: is a drawing illustrating a part of the configuration of the flow analyzer of Configuration Example 1.
- Fig. 9: is a drawing illustrating an example of a coil part of a cooling section in the flow analyzer of Configuration Example 1.
- Fig. 10: is a graph showing a calibration curve created in Comparative Example 3.
- Fig. 11: is a graph showing a calibration curve created in Example 4.

### Description of Embodiments

The following description will discuss embodiments of the present invention in detail. Note, however, that the present invention is not limited to the embodiments, and can be altered within the scope of the matters described herein. The present invention also encompasses, in its technical scope, any embodiment derived by combining, as appropriate, technical means disclosed in differing embodiments. Note that all academic and patent literatures cited in the present specification are incorporated herein by reference. Further, any numerical range expressed as "A to B" in the present specification means "not less than A and not more than B (i.e., a range from A to B which includes both A and B)" unless otherwise specified in the present specification.

### <1> Overview of embodiments of the present invention

In a case where waste water or drinking water is used as a sample to carry out an ICP-MS analysis with respect to a metal other than mercury, the sample is heated and decomposed with nitric acid added thereto (see JIS K102 (2016) 5.1 Pretreatment of sample, The Ministry of Health, Labour and Welfare Notification No. 261, Appended Table 6, 4 Test operations (1) Pretreatment).

In contrast, in a case where the ICP-MS analysis is carried out with respect to mercury, a pretreatment is carried out such that, in a sample solution, the mercury is in stable states of [HgCl₂], [HgCl₃]⁻, and [HgCl₄] ²⁻. In the pretreatment, adding hydrochloric acid to the sample solution binds Hg²⁺ and Cl⁻ in the sample solution. However, in general, a sample sometimes contains hydrogen sulfide, sodium sulfite, an organic matter, zinc, iron, cadmium, and/or the like, and such a substance is a reduction substance. Thus, such a reducing substance causes Hg²⁺ to be Hg⁺ and Hg in the sample solution. Further, in a case where the organic matter is thermally decomposed, the mercury vaporizes. As a result, even in a case where Cl⁻ is added to Hg²⁺ in the sample solution as described above, the reducing substance and the organic matter prevent the mercury from being in the states of [HgCl₂], [HgCl₃]⁻, and [HgCl₄] ²⁻ in the sample solution, so that the mercury vaporizes. This deteriorates a mercury recovery rate.

Mercury has an oxidation-reduction potential of +0.7986. Thus, the inventor of the present invention arrived at a method for analyzing a sample containing mercury in accordance with Embodiment 1 or 2 by finding the following: Mercury can be analyzed with high accuracy in a case where (1) mercury is brought into an oxidation state as a pretreatment and is brought into stable states of [HgCl₂], [HgCl₃]⁻ , and [HgCl₄] ²⁻ by promoting oxidation of the reducing substance and bringing the solution into an oxidation state even in a state of coexistence of reducing substances and (2) the sample solution which has been subjected to the pretreatment is subjected to the ICP-MS analysis while the oxidation state is maintained. The following description will discuss analysis methods in accordance with Embodiments 1 and 2 in detail.

### <2> Method for analyzing sample containing mercury (Embodiment 1)

An analysis method in accordance with Embodiment 1 (hereinafter sometimes referred to as "the present analysis method") includes: a pretreatment step of adding an oxidizer to a sample containing mercury to prepare a sample solution so as to achieve an oxidation-reduction potential of not less than +0.80 V; and an analysis step of subjecting the sample solution which has been prepared in the pretreatment step to a quantitative analysis or a qualitative analysis while maintaining an oxidation state.

According to the present analysis method, by the pretreatment step, the sample solution has an oxidation-reduction potential of not less than +0.80 V, and the mercury is in the oxidation state. Thus, since oxidation of a reducing substance contained in the sample solution is promoted and the solution is in the oxidation state even in a state of coexistence of reducing substances, which are refractory, Hg²⁺ is prevented from being Hg⁺ and Hg in the sample solution. Thus, supply of Cl⁻ to the sample solution brings the mercury into stable states of [HgCl₂], [HgCl₃]⁻, and [HgCl₄] ²⁻. In a case where the sample solution thus pretreated is subjected to a quantitative analysis or a qualitative analysis in the analysis step while the oxidation state is maintained, it is possible to achieve an analysis with a good recovery rate of mercury. That is, the present analysis method makes it possible to use, for example, ICP-MS to analyze, with high accuracy, a sample containing mercury.

In a case where mercury is measured by the foregoing reduction vaporization atomic absorption method, the sample solution that has been pretreated is subjected to a quantitative analysis or a qualitative analysis in a reduced state. Thus, the foregoing reduction vaporization atomic absorption method has a different principle from the present analysis method of subjecting the pretreated sample solution to a quantitative analysis or a qualitative analysis while maintaining the oxidation state.

In the present analysis method, the sample solution has an oxidation-reduction potential of not less than +0.80 V, preferably not less than +1.00 V, more preferably not less than +1.20 V, and even more preferably not less than +1.30 V. Note that, in the present analysis method, the oxidation-reduction potential can be measured with use of a conventionally known oxidation-reduction potential meter (ORP meter).

The oxidizer that is used in the pretreatment step is not particularly limited provided that the oxidizer allows the sample solution to have an oxidation-reduction potential of not less than +0.80 V. The oxidizer is preferably at least any one selected from the group consisting of hypochlorous acid or a salt thereof and permanganic acid or a salt thereof, and is preferably hypochlorous acid or a salt thereof. Hypochlorous acid or a salt thereof and permanganic acid or a salt thereof have oxidizing power sufficient to oxidize mercury. In particular, hypochlorous acid or a salt thereof is an oxidizer that has the highest oxidizing power at normal temperature. In contrast, nitric acid or hydrogen peroxide, which is a common oxidizer, does not have oxidizing power sufficient to solely oxidize mercury. Examples of the salt of the hypochlorous acid include sodium hypochlorite, potassium hypochlorite, calcium hypochlorite, and sodium dichloroisocyanurate. Examples of the salt of the permanganic acid include potassium permanganate, sodium permanganate, ammonium permanganate, silver permanganate, zinc permanganate, magnesium permanganate, calcium permanganate, and barium permanganate.

In the present analysis method, the oxidizer is preferably added in an amount which is excessive relative to the mercury in the sample and the reducing substance contained in the solution. The molar ratio between (a) the mercury in the sample and the reducing substance contained in the solution and (b) the oxidizer is preferably 1:10 to 1:1,000, and more preferably 1:1,000 to 1:100,000. For example, in a case where sodium hypochlorite with 10% effective chlorine is used as the oxidizer, the volume of the amount of the sodium hypochlorite added is preferably not less than 0.002%, and more preferably not less than 0.02%, relative to the volume of the sample containing mercury. In a case where potassium permanganate is used as the oxidizer, the amount of the potassium permanganate added is preferably not less than 2 mg/L, and more preferably not less than 20 mg/L, relative to the volume of the sample containing mercury.

The present analysis method may include a nitric acid-hydrochloric acid addition step of, while maintaining the oxidation state, adding nitric acid, and, if necessary, hydrochloric acid to the sample solution that has been prepared in the pretreatment step. In addition to the oxidizer, nitric acid is further added as an oxidizer. This easily allows the sample solution to have an oxidation-reduction potential of not less than +0.80 V. Note that hydrochloric acid is a supply source for supplying Cl⁻ to the sample solution. In order to achieve a good recovery rate of mercury in the sample, the nitric acid-hydrochloric acid addition step is preferably a step of adding nitric acid and hydrochloric acid. Further, the added amounts of nitric acid and hydrochloric acid can be conventionally known added amounts that are applied in analysis of mercury. In particular, the amount of nitric acid added relative to the volume of the sample is preferably not less than 0.1% and not more than 10%, and more preferably not less than 0.5% and not more than 5%. Similarly, the amount of hydrochloric acid added relative to the volume of the sample is preferably not less than 0.01% and not more than 5%, and more preferably not less than 0.05% and not more than 3%.

A form in which hypochlorous acid or a salt thereof is present differs in accordance with pH. Note, however, that chlorine in hypochlorous acid is present as unstable Cl⁺. In the sample solution, Cl⁺ acquires two electrons so as to be Cl⁻. In a case where an electron donor to Cl⁺ is mercury, the mercury changes from Hg, Hg⁺, to Hg²⁺ and binds to Cl⁻. This brings the mercury into stable states of [HgCl₂], [HgCl₃]⁻, and [HgCl₄] ²⁻ in the sample solution. Thus, in the present analysis method, hypochlorous acid or a salt thereof can function as the oxidizer and also function as the supply source for supplying Cl⁻ to the sample solution.

Therefore, in a case where hypochlorous acid or a salt thereof is used as the oxidizer, in the nitric acid-hydrochloric acid addition step, hydrochloric acid need only be added as necessary, and there can also be a case where hydrochloric acid need not be added. However, in order to achieve a good recovery rate of mercury, nitric acid, hydrochloric acid, and hypochlorous acid or a salt thereof are preferably added to a sample containing mercury.

Further, the present analysis method may further include a heating step of subjecting the sample solution that has been prepared by the pretreatment step or the nitric acid-hydrochloric acid addition step to a heat treatment while maintaining the oxidation state. The heating step results in decomposition of an organic matter contained in the sample. A heating temperature in the heating step only needs to be set such that, for example, the sample has a temperature of not lower than 40°C, preferably not lower than 60°C, and more preferably not lower than 100°C. The upper limit of the heating temperature is preferably not higher than 140°C. A heating time during which the sample is heated is preferably not shorter than 5 minutes, and more preferably not shorter than 15 minutes. The upper limit of the heating time is, for example, not longer than 240 minutes, and more preferably not longer than 120 minutes. In ICP-MS, even in a case where the sample solution contains some organic matter, the organic matter is decomposed during ionization of the sample at 5,000 K to 10,000 K. Thus, analysis can be carried out.

In the present analysis method, the sample solution prepared as described earlier is subjected to a quantitative analysis or a qualitative analysis in the analysis step while the oxidation state is maintained. Note, here, that the analysis includes detection of the presence or absence of an analysis target or measurement of the concentration of the analysis target. Note also that the analysis is not limited to a quantitative analysis or a qualitative analysis. Further, an analysis method is also not particularly limited, and any analysis can be employed. Examples of the analysis method include ICP-MS, inductively coupled plasma optical emission spectrometry (ICP-OES/ICP-AES), atomic absorption photometry, inductively coupled plasma triple quadrupole mass spectrometry, an ion electrode analysis method, and spectrophotometry. Among these, inductively coupled plasma mass spectrometry or inductively coupled plasma optical emission spectrometry is preferable.

In a case where various metals in the sample are simultaneously analyzed by, for example, ICP-MS, a salt of hypochlorous acid used as the oxidizer or permanganic acid or a salt thereof used as the oxidizer sometimes contains a metal serving as the analysis target. Examples of the metal serving as the analysis target include manganese and potassium that are contained in potassium permanganate. Thus, a positive error as much as a metal contained in the oxidizer occurs in a simultaneous analysis of various metals. This requires correction of such an error. In order to carry out an analysis without such an error, a salt of hypochlorous acid or permanganic acid is preferably not a metal salt to be analyzed. In a case where a measurement target does not include sodium, sodium hypochlorite is more preferably used.

A target of the present analysis method is not particularly limited. Examples of the target include various aqueous samples such as tap water, groundwater, seawater, lake water, waste water from factories and the like, ion-exchange water, ultrapure water, steel and nonferrous metals, soils and soil eluates, food, reagents, pharmaceuticals, and reagents for use in semiconductors and industries. The sample that has been decomposed by a pretreatment instrument or device of a closed system or the sample that has been decomposed by a microwave also serves as the target of the present analysis method. The present analysis method brings about an effect of making it possible to analyze mercury with high accuracy in a case where mercury is used as the analysis target to carry out an analysis. The present analysis method can be suitably used for a simultaneous analysis of metal elements including mercury. Therefore, it is possible to simultaneously analyze mercury, which has been difficult to analyze due to volatilization thereof, and the other metal elements.

### <3> Method for using flow analysis method to analyze sample containing mercury (Embodiment 2)

The following description will discuss a flow analysis method in accordance with Embodiment 2. Note that, for convenience, matters which have been already described in <2> will not be described again.

It is known that mercury volatilizes when heated in an open state. Thus, in order to achieve a good recovery rate of mercury, the present analysis method is preferably carried out by a flow analysis method that makes it possible to carry out a heat treatment inside a tube of a closed system. A method for analyzing a sample containing mercury in accordance with Embodiment 2 (hereinafter sometimes referred to as "the present analysis method") differs from Embodiment 1 in that a flow analysis method is used. That is, the analysis method in accordance with Embodiment 2 is carried out by a flow analysis method including: a sample introduction step of introducing, into a tube, a sample containing mercury; and an analysis step of carrying out a quantitative analysis or a qualitative analysis with respect to the sample, the method further including: a pretreatment step and/or a reagent addition step; a heating step; a cooling step; and a gas-liquid separation step. In the present analysis method, a quantitative analysis or a qualitative analysis is carried out with respect to the sample from which gas has been removed in the gas-liquid separation step.

The sample introduction step is a step of introducing the sample into the tube. For example, a sampling device samples a plurality of samples and sequentially and continuously introduces them into the tube at a given flow rate.

The pretreatment step is a step of, prior to the sample introduction step, adding an oxidizer to a sample containing mercury to prepare a sample solution so as to achieve an oxidation-reduction potential of not less than +0.80 V. In the present analysis method, the oxidizer which is used in the pretreatment step is not particularly limited provided that the oxidizer allows the sample solution to have an oxidation-reduction potential of not less than +0.80 V. Examples of the oxidizer that can be used include hypochlorous acid or a salt thereof described earlier and permanganic acid or a salt thereof described earlier. In the present analysis method, the sample solution that is used in the pretreatment step is preferably obtained by adding, to the sample which has been made acidic by nitric acid or made acidic by nitric acid and hydrochloric acid, at least any one of hypochlorous acid or a salt thereof and permanganic acid or a salt thereof. Since the oxidizer used in the pretreatment step, the amount of the oxidizer added, and the amount of hydrochloric acid added as a supply source of Cl⁻ are as described in <2>, a description thereof will be omitted.

The reagent addition step is a step of adding the oxidizer to the sample so as to achieve an oxidation-reduction potential of not less than +0.80 V, the sample being transferred inside the tube. Since the oxidizer which is used in the reagent addition step is similar to those discussed in the description of the pretreatment step and in <2>, a description thereof will be omitted. The reagent addition step may be a step of adding at least the oxidizer to the sample which is transferred inside the tube, and a reagent other than the oxidizer may be added. Note that the oxidizer can be added by a reagent introduction section of a flow analyzer described later.

A method for analyzing a sample containing mercury in accordance with an embodiment of the present invention includes a pretreatment step of, prior to the sample introduction step, adding an oxidizer to the sample containing mercury to prepare a sample solution so as to achieve an oxidation-reduction potential of not less than +0.80 V and/or a reagent addition step of adding the oxidizer to the sample so as to achieve an oxidation-reduction potential of not less than +0.80 V, the sample being transferred inside the tube. That is, prior to the sample introduction step, (i) an oxidizer may be added to the sample containing mercury to prepare a sample solution so as to achieve an oxidation-reduction potential of not less than +0.80 V, (ii) the oxidizer may be added to the sample so as to achieve an oxidation-reduction potential of not less than +0.80 V, the sample being transferred inside the tube, or both (i) and (ii) may be carried out. In particular, in the method for analyzing a sample containing mercury in accordance with an embodiment of the present invention, nitric acid or nitric acid and hydrochloric acid and the oxidizer are more preferably added, at a stage prior to supplying the sample to the sampling device, so as to achieve an oxidation-reduction potential of not less than +0.80 V.

In the present analysis method, at least the pretreatment step among the pretreatment step and the reagent addition step is preferably carried out, and the pretreatment step is preferably carried out prior to the heating step. This is because carrying out the pretreatment step makes it possible to decompose an inorganic compound, an organic matter, and the like, convert a measurement target substance into a solution, and achieve a uniform ratio of the oxidizer to the sample.

The analyzing step is a step of analyzing the sample which is transferred through the tube. Since the analysis step is similar to that described in <2>, a description thereof will be omitted.

The heating step is a step of carrying out a heat treatment with respect to the sample to which the oxidizer has been added. The cooling step is a step of cooling the sample which has been subjected to the heat treatment in the heating step and which is transferred inside the tube. The gas-liquid separation step is a step of removing gas which is present in the tube after cooling in the cooling step. The sample from which the gas has been removed in the gas-liquid separation step is analyzed in the analysis step.

The present analysis method includes the sample introduction step, the pretreatment step and/or the reagent addition step, the heating step, the cooling step, the gas-liquid separation step, and the analysis step, and further includes, between the sample introduction step and the next sample introduction step, a marker introduction step of introducing a marker into the tube. Note, here, that the marker introduction step and the sample introduction step are preferably carried out such that the marker and a given number of samples which given number is one or more are alternately introduced into the tube.

Further, the present analysis method includes a marker detection step of detecting the marker and outputting a detection signal to an analysis device, and in the analysis step, analysis data is acquired on the basis of the detection signal.

The present analysis method may be a method in which a continuous flow analysis method (CFA) is used or may be alternatively a method in which a flow injection analysis method (FIA) is used. In a case where the present analysis method is a method in which the continuous flow analysis method (CFA) is used, the flow analysis method includes a gas bubble segmentation step of producing, in the tube, a plurality of segments that are separated by gas bubbles, by carrying out gas bubble segmentation with respect to the sample and the marker that are introduced into the tube. In a case where the present analysis method is a method in which the flow injection analysis method (FIA) is used, the present analysis method can further include a carrier introduction step prior to the sample introduction step and the marker introduction step.

Further, in the present analysis method, gas bubble segmentation is preferably not carried out after the gas-liquid separation step. With this configuration, the analysis step can be carried out without volatilization of mercury in the sample. Therefore, it is possible to prevent a decrease in analysis accuracy which decrease is caused by volatilization of the mercury.

### <3.1> Flow analyzer for carrying out analysis method in accordance with Embodiment 2 (Configuration Example 1)

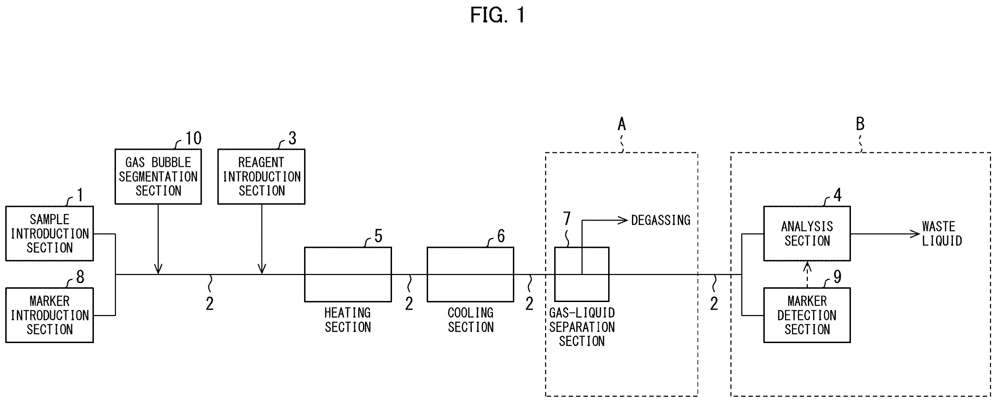

The following description will discuss a flow analyzer for carrying out the analysis method in accordance with Embodiment 2 (hereinafter sometimes referred to as "the present flow analyzer"). Fig. 1 is a drawing schematically illustrating Configuration Example 1 of the present flow analyzer.

The present flow analyzer includes: a sample introduction section 1 that is for introducing a sample into a tube 2; a marker introduction section 8 that is for introducing a marker into the tube 2; a gas bubble segmentation section 10 that produces, inside the tube 2, a plurality of segments separated by gas bubbles, by carrying out gas bubble segmentation with respect to the sample and the marker which have been introduced into the tube 2; a reagent introduction section 3 that adds a reagent to a flow of the sample which is transferred inside the tube 2; a heating section 5 that carries out a heat treatment with respect to the sample which is transferred inside the tube 2 and to which the reagent has been added; a cooling section 6 that cools the sample which has been subjected to the heat treatment; a gas-liquid separation section 7 that removes gas which is present in the tube after cooling; an analysis section 4 that quantitatively or qualitatively analyzes the sample from which the gas has been removed by the gas-liquid separation section 7; and a marker detection section 9 that detects the marker and outputs a detection signal to the analysis section 4. The analysis section 4 acquires analysis data on the basis of the detection signal.

### (Sample introduction section)

The sample introduction section 1 is a device for introducing a sample into the tube 2. For example, the sample introduction section 1 carries out sampling of a sample, and introduces the sample into the tube 2. The sample introduction section 1 includes a thief tube in which the sample is led to the tube 2 and a sampling pump which imparts a suction force to the thief tube. By the sampling pump, the sample is introduced into the tube 2 at a given flow rate. In an embodiment of the present invention, nitric acid or nitric acid and hydrochloric acid and the oxidizer are more preferably added, at a stage prior to supplying the sample to the sampling device, so as to achieve an oxidation-reduction potential of not less than +0.80 V.

### (Marker introduction section)

The marker introduction section 8 is a device for introducing a marker into the tube 2. In an embodiment of the present invention, the marker introduction section 8 includes a thief tube in which the marker is led to the tube 2 and a pump which imparts a suction force to the thief tube. The marker only needs to contain a substance which can be detected by the marker detection section 9, and may be the substance or may be alternatively a solution containing the substance or may be alternatively a dispersion containing the substance.

Note, here, that the marker introduction section 8 and the sample introduction section 1 are configured such that the marker and a given number of samples which given number is one or more are capable of being alternately introduced into the tube 2. That is, the introduction of the marker into the tube 2 by the marker introduction section 8 and the introduction of the given number of samples, which given number is one or more, into the tube 2 by the sample introduction section 1 are alternately switched therebetween. This switching may be carried out manually or may be alternatively carried out automatically.

**In** a case where the marker introduction section 8 and the sample introduction section 1 are configured such that the marker and single samples are capable of being alternately introduced into the tube 2, the marker is introduced into the tube 2 prior to the introduction of each single sample. Then, for each single sample, the marker detection section 9 detects the marker and outputs, to the analysis section 4, a detection signal indicative of the detection of the marker, and the analysis section 4 acquires analysis data on the basis of the detection signal. This makes it possible to stably and continuously measure the sample, because, even in a case where it is not possible to send the sample stably and uniformly in time from a point of the introduction of the sample to the analysis section 4, a difference does not arise between a timing of appearance of a peak in the analysis data and a timing of the acquisition of the analysis data.

Alternatively, in a case where the marker introduction section 8 and the sample introduction section 1 are configured such that the marker and a given number of samples which given number is two or more are capable of being alternately introduced into the tube 2, the marker is introduced into the tube 2 once prior to the introduction of the given number of samples which given number is two or more. Then, the marker detection section 9 detects the marker and outputs, to the analysis section 4, a detection signal indicative of the detection of the marker, and the analysis section 4 sequentially acquires analysis data on the given number of samples on the basis of the detection signal. This makes it possible to stably and continuously measure the samples, because, even in a case where it is not possible to send the samples stably and uniformly in time from the point of the introduction of the samples to the analysis section, a difference between a timing of appearance of a peak in the analysis data and a timing of the acquisition of the analysis data can be reduced to a certain range. The upper limit of the given number may be selected as appropriate, in accordance with the types of the samples, a method of pretreating the samples, and/or the like, and can be, for example, 80, 70, 50, 20, 15, or 10.

The marker which can be detected by the marker detection section 9 is not particularly limited, but is preferably a substance which is not contained in the sample and/or a substance other than a substance to be analyzed. The marker is also preferably a substance which is not decomposed by heat and a reagent which is added between the point of the introduction of the marker into the tube 2 and the marker detection section 9. For example, the marker can be a substance which can be detected by a spectrophotometer. Such a substance is also not particularly limited, and examples thereof include rhodium, palladium, nickel, copper, chromium, manganese, iodine, cobalt, nitrate ion, phosphate ion, and silicate ion. Alternatively, the above substance can be a substance which can be detected by a voltammeter. Such a substance is also not particularly limited, and examples thereof include copper, cadmium, nickel, mercury, arsenic, and selenium. Alternatively, the above substance can be a substance which can be detected by an ion electrode meter. Such a substance is also not particularly limited, and examples thereof include calcium, potassium, fluorine, and ammonia. Alternatively, the above substance can be a substance which can be detected by an ion chromatography. Such a substance is also not particularly limited, and examples thereof include ions of inorganic acid and ions of organic acid, phenol, hydrazine, amino acid, and polysaccharides. Alternatively, the above substance can be a substance which can be detected by a turbidimeter. Such a substance is also not particularly limited, and examples thereof include silica which is a fine particulate substance that is not dissolved by acid other than hydrofluoric acid. Alternatively, the above substance can be a substance which can be detected by a fluorophotometer. Such a substance is also not particularly limited, and examples thereof include benzene, coumarin, and naphthalene. Among these substances, rhodium, palladium, cobalt, nickel, copper, and the like are particularly preferable as the marker, from the viewpoint of easiness of detection.

### (Gas bubble segmentation section)

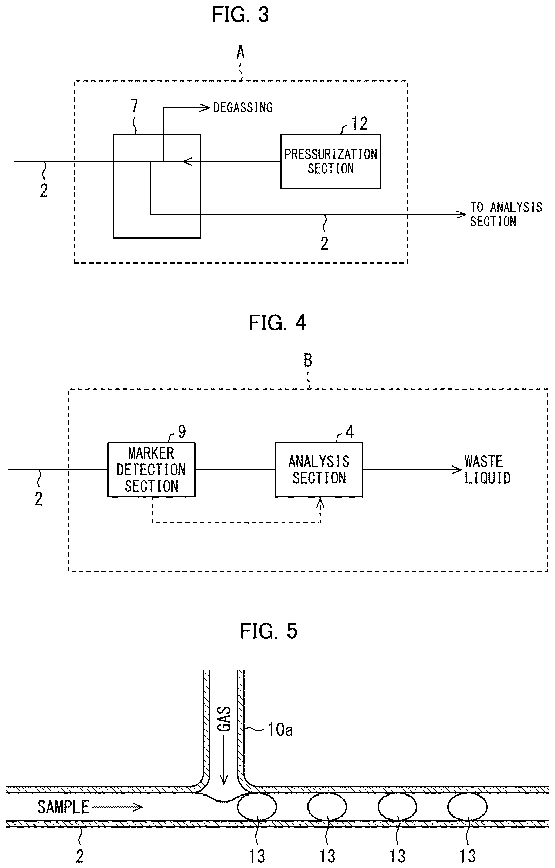

The gas bubble segmentation section 10 is a device for producing, inside the tube 2, a plurality of segments separated by gas bubbles 13, by carrying out gas bubble segmentation with respect to the sample and the marker which have been introduced into the tube 2, as illustrated in Fig. 5. In an embodiment of the present invention, the gas bubble segmentation section 10 includes a gas introduction tube 10a in which gas is led to the tube 2 and a gas introduction pump (not illustrated) which imparts a suction force to the gas introduction tube. By carrying out the gas bubble segmentation, it is possible to suitably mix the reagent and the like, due to a turbulent flow in a segmented liquid which is divided by the gas bubbles 13. Since the segmented liquid, i.e., the sample which forms each of the segments that are separated by the gas bubbles 13, is divided by the gas bubbles 13 and independently flows inside the tube 2, it is possible to prevent interdiffusion of the sample. The gas for the gas bubble segmentation is preferably air, but may be an inert gas(es) such as argon and/or helium. Various gases such as nitrogen and oxygen can be also used. Each of these gases may be used solely or two or more of these gases may be mixed and used. A method of, in this manner, introducing a reagent into a continuous flow of a sample inside a tube which sample is segmented by gas bubbles, carrying out a reaction operation, removing the gas bubbles, and then carrying out an analysis in a detector which is provided downstream is referred to as a continuous flow analysis method (CFA). Note that, in the present specification, the term "downstream" means being downstream of a flow of a sample inside a tube for a flow analysis.

### (Reagent introduction section)

The reagent introduction section 3 is a device for adding the reagent to the flow of the sample that is transferred inside the tube 2. In the reagent introduction section 3, the foregoing oxidizer may be added to the sample so as to achieve an oxidation-reduction potential of not less than +0.80 V, the sample being transferred inside the tube 2. The reagent introduction section 3 includes a reagent introduction tube in which the reagent is led to the tube 2 and a reagent introduction pump which imparts a suction force to the reagent introduction tube. The reagent can be a reagent which is added during the pretreatment of the sample. The reagent is not particularly limited, and examples thereof include: acid such as nitric acid, hydrochloric acid, sulfuric acid, perchloric acid, phosphoric acid, and hydrofluoric acid; stabilizers for mercury, such as L-cysteine and gold; and alkali such as sodium hydroxide, potassium hydroxide, sodium peroxide, calcium carbonate, and sodium carbonate. Each of these reagents may be used solely or two or more of these reagents may be used in combination. In a case where two or more types of reagents are used, a plurality of reagent introduction sections 3 may be provided. Alternatively, in a case where at least two types of reagents, out of the two or more types of reagents, can be mixed and then introduced, the reagents which can be mixed may be mixed and then introduced from a single reagent introduction section 3. The reagent may be selected, as appropriate, in accordance with an analysis target, an analysis method, and the like, but more preferably contains an oxidizer that is at least any one selected from the group consisting of hypochlorous acid or a salt thereof and permanganic acid or a salt thereof, in order to analyze mercury with high accuracy. In a case where the inductively coupled plasma mass spectrometry (ICP-MS) is used as the analysis method, the reagent preferably contains hypochlorous acid or a salt thereof. In a case where nitric acid or nitric acid and hydrochloric acid, the oxidizer, and the like are added to a standard solution and a measurement sample in advance, a reagent solution containing equivalent acid, an oxidizer, and the like is preferably allowed to flow as a buffer to the reagent introduction section 3. Alternatively, the reagent introduction section 3 need not be provided.

### (Heating section)

The heating section 5 is a device for carrying out a heat treatment with respect to the sample that is transferred inside the tube 2. The heating section 5 can be a thermostatic bath which includes a heater. Note, however, that a configuration of the heating section 5 is not limited to such a configuration, and the heating section 5 may be an ultrasonic decomposition device, a microwave, an autoclave decomposition device, or the like. In the heating section 5, the tube 2 may form, for example, a coil. In an embodiment of the present invention, the heating section 5 is provided downstream of the reagent introduction section 3. By heating the sample to which the reagent has been added, it is possible to promote a reaction between the sample and the reagent and thereby pretreat the sample. In the heating section 5, it is possible to carry out, for example, thermolysis or high-temperature and high-pressure decomposition of the sample.

A heating temperature in the heating section 5 only needs to be set such that, for example, the sample has a temperature of not lower than 50°C, not lower than 60°C, not lower than 70°C, or not lower than 80°C. The upper limit of the heating temperature is preferably not higher than 140°C, not higher than 130°C, or not higher than 120°C. A heating time during which the sample is heated is, for example, not shorter than 5 minutes, not shorter than 10 minutes, or not shorter than 20 minutes per unit of the sample introduced (a single sample). The upper limit of the heating time is, for example, not longer than 80 minutes, and more preferably not longer than 40 minutes. By setting the heating temperature and the heating time in the heating section 5 to fall within the above respective ranges, it is possible to sufficiently decompose a compound, including a mercury compound, in the sample.

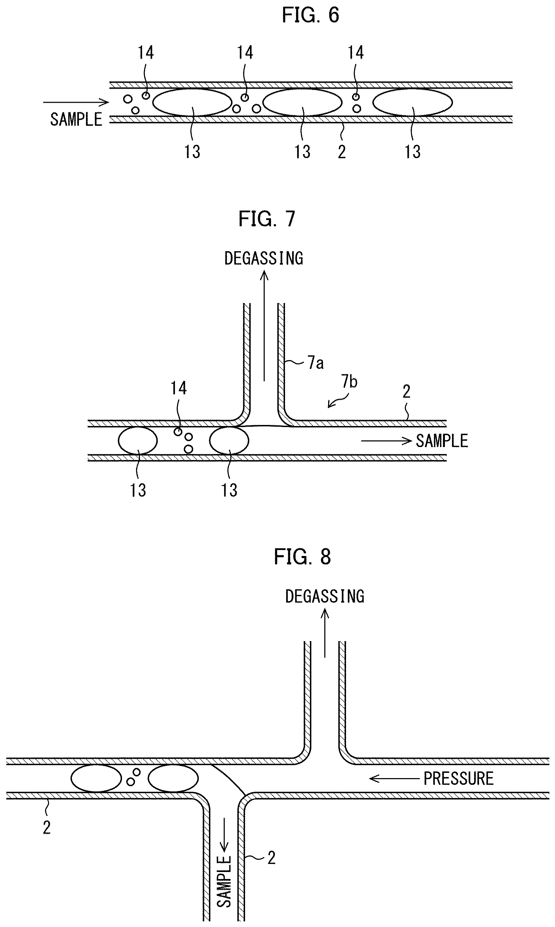

Fig. 6 is a drawing schematically illustrating the gas bubbles inside the tube after the heat treatment in the present flow analyzer. As illustrated in Fig. 6, the gas bubbles 13 which have been introduced by the gas bubble segmentation section 10 expand by the heat treatment. The reaction between the sample and the reagent may cause generation of gas, and, in this case, the gas thus generated also expand by the heat treatment. The gas which has been generated by the reaction and which has expanded disperse as gas bubbles 14 in the segmented liquid or unite with the gas bubbles 13 which have been introduced by the gas bubble segmentation section 10. At least a part of mercury contained in the sample at this time sometimes vaporizes by the heat treatment, and sometimes moves into the gas phase of the gas bubbles 13 or the gas bubbles 14.

In an example illustrated in Fig. 1, the flow analyzer includes a pretreatment unit which is constituted by a single reagent introduction section 3 and a single heating section 5 that is provided downstream of the single reagent introduction section 3. Alternatively, instead of the single reagent introduction section 3, a plurality of reagent introduction sections 3 may be provided upstream of the heating section 5 so that a plurality of reagents are added. Note that the pretreatment unit in the present configuration example is not a unit for carrying out "a pretreatment step of, prior to the sample introduction step, adding an oxidizer to the sample containing mercury to prepare a sample solution" described in <3>.

In the example illustrated in Fig. 1, the flow analyzer includes the pretreatment unit which is constituted by the single reagent introduction section 3 and the single heating section 5 that is provided downstream of the single reagent introduction section 3. Alternatively, provided may be one or more reagent introduction sections 3, a single heating section 5 that is located downstream of the one or more reagent introduction sections 3, and a reagent introduction section 3 that is located downstream of the single heating section 5 and upstream of the analysis section 4. The reagent introduction section 3 that is located downstream of the single heating section 5 and upstream of the analysis section 4 may add, for example, nitric acid, hydrochloric acid, or nitric acid and hydrochloric acid as the reagent. In a case where the inductively coupled plasma mass spectrometry (ICP-MS) is used, it is possible to more stably measure mercury by adding hydrochloric acid to a nitric acid solution at a point that is located upstream of the heating section 5 or at a point that is located downstream of the heating section 5 and upstream of the analysis section 4.

Further, in the example illustrated in Fig. 1, the flow analyzer includes a single pretreatment unit. Alternatively, the flow analyzer may include a plurality of pretreatment units, and, in this case, each of the plurality of pretreatment units may include a different number of reagent introduction sections 3. In a case where the flow analyzer includes a plurality of pretreatment units, it is possible to carry out a pretreatment which involves, for example, carrying out acidolysis under heating, adding acid again, and then carrying out acidolysis under heating. In a case where the flow analyzer includes a plurality of reagent introduction sections 3, reagents which are added by the plurality of reagent introduction sections 3 may be identical to or different from each other.

In the example illustrated in Fig. 1, the reagent introduction section 3 is provided downstream of the gas bubble segmentation section 10. Note, however, that disposition of the reagent introduction section 3 is not limited such disposition. The reagent introduction section 3 may be provided upstream of the gas bubble segmentation section 10. In a case where the flow analyzer includes a plurality of reagent introduction sections 3, the plurality of reagent introduction sections 3 may be provided upstream and downstream of the gas bubble segmentation section 10. Alternatively, there is also a case where the reagent which is introduced by the reagent introduction section 3 is a reagent for allowing the detection of the marker (e.g. coloring liquid). In such a case, the reagent introduction section 3 may be provided downstream of the heating section 5. Alternatively, one or more reagent introduction sections 3 each of which is for introducing the reagent that is used in the pretreatment may be provided upstream of the heating section 5, and one or more reagent introduction sections 3 each of which is for introducing the reagent that is used to allow the detection of the marker may be provided downstream of the heating section 5.

### (Cooling section)

The cooling section 6 is a device that cools the sample which has been subjected to the heat treatment by the heating section 5 and which is transferred inside the tube 2. The cooling section 6 preferably includes a tube which has a length of 0.5 m to 3.0 m and inside which the sample is transferred. The tube 2 is connected to both ends of the tube of the cooling section 6. The sample which has been subjected to the heat treatment by the heating section 5 and which is transferred inside the tube 2 is, as it is, continuously transferred inside the tube of the cooling section 6. The sample which has passed through the tube of the cooling section 6 continues to be continuously transferred inside the tube 2. In other words, the tube of the cooling section 6 serves as the tube 2. The tube 2 and each of the both ends of the tube of the cooling section 6 are connected to each other such that the sample and the gas which are transferred inside the tube are sealed in the tube. The inner diameter of the tube 2 and the inner diameter of the tube of the cooling section 6 may be equal to or different from each other, provided that the sample can be continuously transferred.

The sample which has been subjected to the heat treatment by the heating section 5 and which is transferred inside the tube 2 is introduced into the tube of the cooling section 6, and cooled while being transferred inside the tube of the cooling section 6. The length of the tube of the cooling section 6 is more preferably 0.7 m to 2.5 m, and even more preferably 1.0 m to 2.0 m. The length of the tube of the cooling section 6 is preferably not shorter than 0.5 m, because the sample which has been subjected to the heat treatment is sufficiently cooled while being transferred inside the tube of the cooling section 6. In a conventional flow analyzer which includes a gas-liquid separation section that is located downstream of a heating section, the length of a tube from the heating section to the gas-liquid separation section is typically 0.5 cm to 20 cm. In an analysis in which an analysis target is not mercury, it is not necessary to make the tube from the heating section to the gas-liquid separation section long. This is because there is not a problem that an element to be measured is removed together with gas even in a case where the gas is removed in a state where a sample inside the tube has a high temperature. In a case where mercury is to be measured and in a state where the temperature inside the tube is high, the mercury is contained in a gas phase. Therefore, by decreasing the temperature inside the tube so that the mercury is brought back to a liquid phase, it is possible to prevent the mercury from being removed together with the gas. Further, in a case where the length of the tube of the cooling section 6 is not longer than 3 m, time taken to transfer the sample is not too long. Therefore, it is possible to shorten the time needed for an analysis, and possible to make the device compact.

A cooling method in the cooling section 6 is not particularly limited, but examples thereof include: a method in which the tube 2 inside which the sample that has been subjected to the heat treatment is transferred is cooled by air; and a method in which the tube 2 is cooled by a cooling medium such as water (for example, a method in which the tube 2 is cooled by water, and a method in which the tube 2 is cooled by ice water).

The material of the tube of the cooling section 6 is not particularly limited, provided that the material is inactive with respect to the sample which is transferred inside the tube. Examples of the material include fluorine-based resins such as perfluoroalkoxyalkane (PFA) and polytetrafluoroethylene (PTFE); olefin-based resins such as polypropylene and polyethylene; glass; and PEEK resin (polyether ether ketone resin). Note that the material of the tube 2 in the present flow analyzer is also not particularly limited, provided that the material is inactive with respect to the sample which is transferred inside the tube. For example, a material similar to that of the tube of the cooling section 6 as described above can be used.

The inner diameter of the tube of the cooling section 6 is, but is not limited to, preferably 0.5 mm to 3.0 mm, more preferably 1.2 mm to 2.5 mm, even more preferably 1.5 mm to 2.3 mm, particularly preferably 1.8 mm to 2.2 mm, and most preferably 1.9 mm to 2.1 mm. In a case where the inner diameter is not less than 0.5 mm, it is possible to transfer the sample inside the tube at a moderate rate. In a case where the inner diameter is not more than 3.0 mm, the sample which is transferred inside the tube is more frequently brought into contact with a wall of the tube, so that it is possible to more rapidly cool the sample. Therefore, such an inner diameter is preferable.

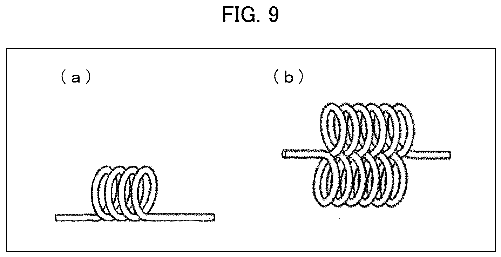

The shape of the tube of the cooling section 6 is not particularly limited, and may be a linear shape or a curved shape. In particular, the tube preferably includes a coil part. The coil part has a shape of, for example, a helix, a figure-eight helix, or the like. Fig. 9 schematically illustrates examples of the coil part. (a) of Fig. 9 illustrates the coil part having a shape of a helix. (b) of Fig. 9 illustrates the coil part having a shape of a figure-eight helix. In a case where the tube of the cooling section 6 includes the coil part, the sample transferred inside the tube is transferred while being circulated inside the tube. This causes the sample to be stirred and mixed. Therefore, at the same time the sample is cooled, mercury in the gas phase is more likely to transition to the liquid phase. Thus, such a configuration is preferable, because it is possible to prevent a loss caused by vaporization of mercury and carry out an accurate analysis.

In a case where the tube of the cooling section 6 includes the coil part, the tube may be constituted solely by the coil part or may alternatively include the coil part and a linear part. From the viewpoint of carrying out a more accurate analysis, the tube more preferably includes the coil part and also the linear part. The reason therefor is not clear, but the following inference is derived. That is, since the sample is stirred and mixed in the coil part, the coil part has the function of cooling the sample and, at the same time, transitioning mercury in the gas phase to the liquid phase. Meanwhile, the linear part has the function of more efficiently cooling the sample. Therefore, by combining both the parts, it is possible to more effectively prevent a loss caused by vaporization of mercury. The disposition of the coil part and the linear part can be, but is not limited to, for example, such that the coil part is disposed downstream of the linear part. According to this configuration, it is considered that (i) the sample which has been subjected to the heat treatment is cooled mainly in the linear part and thereby mercury which has been present as gas in the sample becomes liquid and then (ii) in the coil part, the sample is stirred and mixed and the mercury transitions to the liquid phase. In such a case, the ratio between the length of the linear part of the tube and the length of the coil part of the tube is, for example, 1:1 to 10:1, and more preferably 2:1 to 5:2. Note, here, that the "length of the coil part" in the expression "ratio between the length of the linear part of the tube and the length of the coil part of the tube" is intended to be the length of the tube which forms the coil part, that is, the length of the tube in a case where the coil part is stretched linearly. In the configuration in which the coil part is disposed downstream of the linear part, a second linear part may be further present downstream of the configuration in which the coil part is disposed downstream of the linear part. In such a case, the ratio between the length of the linear part of the tube and the length of the coil part of the tube is intended to be the ratio between the length of the linear part which is located upstream of the coil part and the length of the coil part, and the length of the second linear part is not taken into consideration.

Alternatively, the configuration in which the coil part is disposed downstream of the linear part may be repeated a plurality of times, for example, 2 to 10 times. Also in such a case, the "length of the coil part" is as described above. A linear part which serves as the last stage may be further present downstream of a configuration in which the configuration in which the coil part is disposed downstream of the linear part is repeated a plurality of times. **In** such a case, the ratio between the length of the linear part of the tube and the length of the coil part of the tube is intended to be the ratio between the total length of the linear parts which are located upstream of the respective plurality of coil parts and the total length of the plurality of coil parts, and the length of the linear part at the last stage is not taken into consideration.

The number of turns of the helix, the figure-eight helix, and the like of the coil part is also not particularly limited. The number of turns is, for example, 1 turn to 20 turns, more preferably 2 turns to 10 turns, and even more preferably 4 turns to 7 turns. The number of turns is preferably not less than 1 turn, because the sample is stirred and mixed more uniformly and, therefore, at the same time the sample is cooled, mercury in the gas phase is more likely to transition to the liquid phase. The number of turns is preferably not more than 20 turns, because it is possible to make the device compact and shorten the time needed for an analysis.

In a case where the coil part has, for example, a shape of a helix, the outer diameter of the helix (referred to as "coil diameter") is, but is not particularly limited to, for example, 10 mm to 70 mm, more preferably 15 mm to 60 mm, even more preferably 20 mm to 50 mm, and most preferably 25 mm to 40 mm. The coil diameter is preferably not less than 10 mm, because it is possible to stably send the liquid. Further, in a case where the coil diameter is not more than 70 mm, the liquid can be mixed more properly. Therefore, a coil diameter of not more than 70 mm is preferable.

In a case where the coil part has, for example, a shape of a helix, the coil pitch of the helix is, but is not particularly limited to, for example, 0.7 mm to 40 mm, more preferably 0.8 mm to 30 mm, and even more preferably 0.9 mm to 25 mm. The coil pitch is preferably not less than 0.7 mm, because it is possible to form the coil part in which a tube having a preferable tube diameter is wound. Further, the coil pitch is preferably not more than 40 mm, because the coil part having such a coil pitch has a size with which the coil part does not take up much space.

In a case where the coil part has, for example, a shape of a figure-eight helix, the maximum length of the figure eight of the figure-eight helix is similar to the coil diameter in the case of the helix, and the pitch of the figure-eight helix is similar to the coil pitch in the case of the helix.

A cooling temperature in the cooling section 6 only needs to be set such that, for example, the sample has a temperature of not higher than 30°C, preferably not higher than 20°C, and more preferably not higher than 10°C. A cooling time during which the sample is cooled is not particularly limited, provided that the sample can be sufficiently cooled. The cooling time is, for example, not shorter than 2 minutes, not shorter than 3 minutes, or not shorter than 5 minutes per unit of the sample introduced. The upper limit of the cooling time is, for example, 15 minutes per unit of the sample introduced. By setting the cooling temperature and the cooling time in the cooling section 6 so as to fall within the above respective ranges, it is possible to form, into liquid, mercury which is present as gas in the sample.

The cooling section 6 is disposed between the heating section 5 and the gas-liquid separation section 7. The sample and the gas bubbles 13 and 14 which have been cooled by the cooling section 6 and which are transferred inside the tube 2 are transferred inside the tube 2 to the gas-liquid separation section 7.

### (Gas-liquid separation section)

The gas-liquid separation section 7 is a device for sequentially removing the gas which is present inside the tube 2. Fig. 7 is a drawing schematically illustrating how the gas which is present inside the tube 2 is removed by the gas-liquid separation section 7. As illustrated in Fig. 7, the gas-liquid separation section 7 includes: a three-way tube 7b that includes (i) a tube (tube 2) inside which the sample continues to be transferred in a horizontal direction or a downward direction in a downstream direction of the tube 2 and (ii) a degassing tube 7a which branches upward; a pump (not illustrated) that imparts a suction force to the tube 2 inside which the sample continues to be transferred in the horizontal direction or the downward direction; and a degassing pump (not illustrated) that imparts a suction force to the degassing tube.

As illustrated in Fig. 7, the gas which is present in the tube 2 (gas bubbles 13 and 14) rises upward, and is therefore removed (degassing) through the degassing tube of the three-way tube which degassing tube branches upward.

### (Analysis section)

The analysis section 4 is a device which analyzes the sample to which the reagent has been added, which has been subjected to the heat treatment by the heating section 5, which has been cooled by the cooling section 6 after the heat treatment, and from which the gas has been removed by the gas-liquid separation section 7 after the cooling. In an embodiment of the present invention, the analysis section 4 can be an inductively coupled plasma mass spectrometer (ICP-MS). Note, however, that the analysis section 4 is not limited to such an analysis device, and may be any analysis device. The analysis section 4 may be, for example, an inductively coupled plasma optical emission spectrometer (ICP-OES), an atomic absorption photometer, an inductively coupled plasma triple quadrupole mass spectrometer, an ion electrode meter, or a spectrophotometer. The analysis section 4 is not limited to a device for detecting the presence or absence of mercury or measuring only the concentration of the mercury, and is also preferably a device which can collectively measure the other metal elements. Note also that the analysis is not limited to a quantitative analysis or a qualitative analysis.

### (Marker detection section)

Between the gas-liquid separation section 7 and the analysis section 4, the marker detection section 9 is connected to a branch tube which is for extracting, from the tube 2, the sample and the marker which sequentially flow inside the tube 2. In other words, the tube 2 branches into two at a stage after the removal of the gas by the gas-liquid separation section 7 and prior to introduction of the sample into the analysis section 4, and one (referred to as "tube 2" even after the branching) is connected to the analysis section 4 while the other (referred to as "branch tube") is connected to the marker detection section 9. The marker detection section 9 continuously measures the liquid extracted from the tube 2 through the branch tube. When the marker detection section 9 detects the marker, the marker detection section 9 outputs the detection signal to the analysis section 4. Then, in an embodiment of the present invention, upon receipt of the detection signal, the analysis section 4 starts acquiring the analysis data. In an embodiment of the present invention, the marker detection section 9 is a spectrophotometer. In a case where the marker contains rhodium, the marker detection section 9 outputs, to the analysis section 4, the detection signal when the marker detection section 9 detects the rhodium. Note, however, that the marker detection section 9 is not limited to the spectrophotometer, and can be, for example, a voltammeter, an ion electrode meter, an ion chromatograph, a turbidimeter, or a fluorophotometer. The marker and the sample which sequentially flow are introduced into the analysis section 4 and the marker detection section 9 at the same timing through the tube 2 and the branch tube, respectively. Alternatively, the marker and the sample which sequentially flow may be introduced into the analysis section 4 and the marker detection section 9 at different timings, not at the same timing. In a case where the marker and the sample which sequentially flow are introduced into the analysis section 4 and the marker detection section 9 at different timings, the marker and the sample need to be introduced into the marker detection section 9 at a timing earlier than a timing at which they are introduced into the analysis section 4. In the above embodiment, the analysis section 4 is configured to start acquiring the analysis data upon receipt of the detection signal. However, the analysis section 4 may be set to start acquiring the analysis data a given time after the receipt of the detection signal.

In an embodiment of the present invention, the analysis section 4 and the marker detection section 9 are disposed in parallel as described above. Note, however, that the analysis section 4 and the marker detection section 9 may be disposed in series as in an embodiment illustrated in Fig. 4. In the embodiment illustrated in Fig. 4, the marker detection section 9 is disposed between the gas-liquid separation section 7 and the analysis section 4. At the stage after the removal of the gas by the gas-liquid separation section 7 and prior to the introduction of the sample into the analysis section 4, the marker detection section 9 continuously measures the liquid that is introduced thereinto through the tube 2, and outputs the detection signal to the analysis section 4 when the marker detection section 9 detects the marker. Then, after the receipt of the detection signal, the analysis section 4 starts acquiring the analysis data. In this case, it is only necessary to adjust a timing of a start of the acquisition of the analysis data so that the analysis section 4 can measure the sample which has reached the analysis section 4. For example, the analysis section 4 may be set to start acquiring the analysis data a given time after the receipt of the detection signal.

In an example illustrated in Fig. 1, the sample from which the gas has been removed by the gas-liquid separation section 7 is transferred to the marker detection section 9 or the analysis section 4 which is located downstream of the gas-liquid separation section 7. Between the gas-liquid separation section 7 and the marker detection section 9 or the analysis section 4, a mixing coil for further mixing the sample may be provided. The mixing coil is the tube 2 which is formed in a shape of a coil, and when the sample passes through the mixing coil, the sample which flows inside the tube 2 is mixed. The shape of the mixing coil is not particularly limited, but can be a shape that is similar to any of the shapes illustrated in Fig. 9.

In the flow analyzer in accordance with Embodiment 1 described above, the sample is continuously introduced into the tube, the gas bubble segmentation is carried out, the reagent is introduced, a reaction is promoted by the heating section, mercury which has vaporized is transitioned to the liquid phase by cooling the sample and the gas bubbles by the cooling section, and then the gas is removed by the gas-liquid separation section. This makes it possible to prevent a loss caused by vaporization of mercury and continuously measure the analysis data by the analysis section. Furthermore, the flow analyzer includes: the marker introduction section which is for introducing the marker into the tube; and the marker detection section which detects the marker and outputs the detection signal to the analysis section, and the analysis section acquires the analysis data on the basis of the detection signal. This makes it possible to stably and continuously measure the sample.

In general, in a case where a flow analyzer includes a gas-liquid separation section, a gas bubble segmentation device which newly leads gas to a tube is often provided downstream of the gas-liquid separation section. In the present flow analyzer, a gas bubble segmentation device which newly leads gas to the tube is not provided between the gas-liquid separation section and the analysis section. With this configuration, mercury in the sample is introduced into the analysis section 4 without volatilizing. Therefore, it is possible to prevent a decrease in analysis accuracy which decrease is caused by volatilization of the mercury.

The present flow analyzer may include a pressurization section which applies, to the heating section 5 from downstream of the heating section 5, pressure against the flow of the sample. Figs. 3 and 8 are each a partial schematic drawing illustrating an example of the flow analyzer which includes a pressurization section 12 that applies pressure against the flow of the sample, from downstream of the heating section 5 or downstream of the cooling section 6. In the example illustrated in each of Figs. 3 and 8, pressure against the flow of the sample is applied from the pressurization section 12 which is provided downstream of the gas-liquid separation section. The pressurization section 12 includes, for example, a compressor and a valve. By providing the pressurization section, it is possible to decompose the sample at high temperature and high pressure in the heating section 5. Therefore, the flow analyzer preferably includes the pressurization section, because it is possible to efficiently decompose an impurity including an organic matter, etc. and dissolve mercury in a solution. The pressure applied by the pressurization section 12 is not particularly limited, and may be selected, as appropriate, in accordance with the heating temperature, the heating time, and/or the like in the heating section 5. For example, the pressure is not more than 0.14 MPa, and, in some cases, includes a negative pressure of less than 0.1 MPa. The pressure is more preferably more than 0.1 MPa and not more than 0.13 MPa.

In the present flow analyzer, an autosampler can be used as the sample introduction section. Further, an ultrasonic homogenizer or a stirrer may be provided so as to pulverize and/or stir the sample prior to the sampling.

The present flow analyzer may further include a dilution section which is provided in the middle of the tube. This makes it possible to automatically carry out desired dilution in the flow analyzer, in a case where it is necessary to dilute the sample depending on the concentration of the sample. As such a dilution section, a commercially available automatic dilution device can be suitably used.

Further, the present flow analyzer preferably adds nitric acid or nitric acid and hydrochloric acid and an oxidizer to a standard solution and a measurement sample in advance. Adhesion of mercury to a tube sometimes occurs. Thus, adding an oxidizer to a standard solution and a sample to each of which nitric acid or nitric acid and hydrochloric acid has/have been added in advance makes it possible to prevent the adhesion. A preparation device which, as a pretreatment, adds an oxidizer to a sample containing mercury to prepare a sample solution so as to achieve an oxidation-reduction potential of not less than +0.80 V may be incorporated in the sample introduction section or provided upstream of the sample introduction section. In this case, the oxidizer which is added to the adjustment device is not particularly limited provided that the oxidizer allows the sample solution to have an oxidation-reduction potential of not less than +0.80 V. Examples of the oxidizer which can be used include hypochlorous acid or a salt thereof and permanganic acid or a salt thereof. That is, the oxidizer which is added to the adjustment device is preferably at least any one of hypochlorous acid or a salt thereof and permanganic acid or a salt thereof. In the present flow analyzer, a device which pretreats the sample that is not a liquid but a solid or the like and which thereby prepares a liquid sample may be incorporated in the sample introduction section or provided upstream of the sample introduction section. The flow analyzer is a device which analyzes a liquid sample by a flow analysis method. Thus, it is not possible to measure, as it is, the sample that is not a liquid but a solid or the like. Therefore, by providing the device which pretreats the sample that is not a liquid but a solid or the like and which thereby prepares a liquid sample, it is possible to unin-termittedly carry out steps from the pretreatment of the sample that is not a liquid but a solid or the like to the analysis. As such a device, a device which fully automatically pretreats the sample that is not a liquid but a solid or the like is more preferable. For example, a fully automatic acidolysis pretreating device which fully automatically carries out addition of the reagent, mixing, heating, and dilution in a measuring flask can be suitably used.

### <3.2> Flow analyzer for carrying out analysis method in accordance with Embodiment 2 (Configuration Example 2)

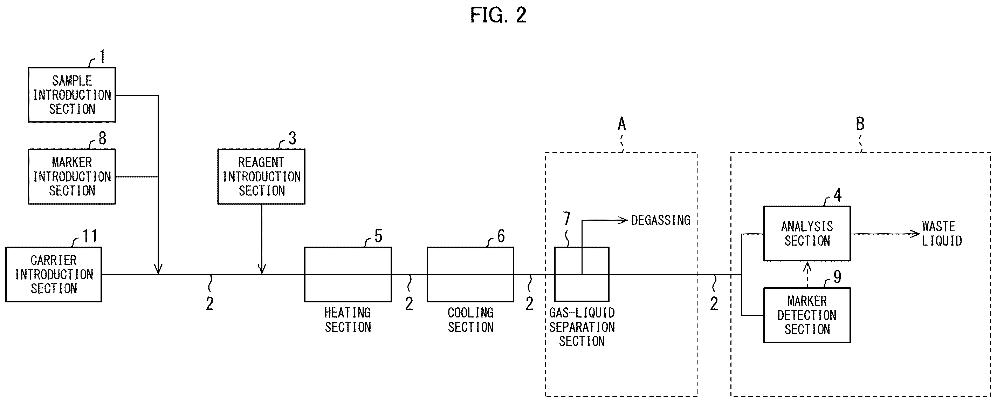

The following description will discuss another Configuration Example 2 of the present flow analyzer. Fig. 2 is a drawing schematically illustrating Configuration Example 2 of the present flow analyzer. Note that, for convenience, members which have the same functions as those of the members that have been described in <3.1> will be given the same reference signs and will not be described again.

The flow analyzer of Configuration Example 2 employs a flow injection analysis method (FIA) in which a reagent is introduced into a flow of a sample that is inside a tube and that is not segmented by gas bubbles, a reaction operation is carried out, and then an analysis is carried out by a detector that is provided downstream.

A flow analyzer of Configuration Example 1 includes: a carrier introduction section 11 that introduces a carrier into a tube 2; a sample introduction section 1 that is for introducing a sample into a flow of the carrier inside the tube 2; a marker introduction section 8 that is for introducing a marker into the flow of the carrier inside the tube 2; a reagent introduction section 3 that adds a reagent to a flow of the sample which is transferred inside the tube 2; a heating section 5 that carries out a heat treatment with respect to the sample which is transferred inside the tube 2 and to which the reagent has been added; a cooling section 6 that cools the sample which has been subjected to the heat treatment; a gas-liquid separation section 7 that removes gas which is present in the tube after cooling; an analysis section 4 that quantitatively or qualitatively analyzes the sample from which the gas has been removed by the gas-liquid separation section 7; and a marker detection section 9 that detects the marker and outputs a detection signal to the analysis section 4. The analysis section 4 acquires analysis data on the basis of the detection signal.

The flow analyzer of Configuration Example 2 has the same configuration as that of the flow analyzer illustrated in Fig. 1, except that, in the former, the carrier introduction section 11 is provided upstream of the sample introduction section 1 which is for introducing the sample to the tube 2 and the marker introduction section 8 which is for introducing the marker into the tube 2, and a gas bubble segmentation section is not provided.

The flow analyzer of Configuration Example 2 employs the flow injection analysis method (FIA).

As such, the carrier introduction section 11 introduces the carrier into the tube 2, and the sample introduction section 1 and the marker introduction section 8 introduce the sample and the marker, respectively, into the flow of the carrier inside the tube 2.

The carrier is not particularly limited, provided that the carrier is a liquid which does not adversely affect a pretreatment and the analysis of the sample. Examples thereof include water, surfactants, acid solutions, and alkaline solutions.

The other configurations of the flow analyzer of Configuration Example 2 are as described in Configuration Example 1, and therefore description thereof will be omitted.

### <4> Summary

Embodiments of the present invention include the following features.
<1> A method for analyzing a sample containing mercury, including: a pretreatment step of adding an oxidizer to a sample containing mercury to prepare a sample solution so as to achieve an oxidation state in which an oxidation-reduction potential is not less than +0.80 V; and an analysis step of subjecting the sample solution which has been prepared in the pretreatment step to a quantitative analysis or a qualitative analysis while maintaining the oxidation state.
<2> A method for analyzing a sample containing mercury, the method being carried out by a flow analysis method including: a sample introduction step of introducing, into a tube, a sample containing mercury; and an analysis step of carrying out a quantitative analysis or a qualitative analysis with respect to the sample, the method including: a pretreatment step of, prior to the sample introduction step, adding an oxidizer to the sample containing mercury to prepare a sample solution so as to achieve an oxidation-reduction potential of not less than +0.80 V and/or a reagent addition step of adding the oxidizer to the sample so as to achieve an oxidation-reduction potential of not less than +0.80 V, the sample being transferred inside the tube; a heating step of carrying out a heat treatment with respect to the sample to which the oxidizer has been added; a cooling step of cooling the sample which has been subjected to the heat treatment and which is transferred inside the tube; and a gas-liquid separation step of removing gas which is present in the tube after cooling, a quantitative analysis or a qualitative analysis being carried out with respect to the sample from which the gas has been removed in the gas-liquid separation step.
<3> The method as described in <1> or <2>, wherein the oxidizer is at least any one selected from the group consisting of hypochlorous acid or a salt thereof and permanganic acid or a salt thereof.
<4> The method as described in <2> or <3>, further including a gas bubble segmentation step of producing, inside the tube, a plurality of segments separated by gas bubbles, by carrying out gas bubble segmentation with respect to the sample which is introduced into the tube.
<5> The method as described in any one of <2> to <4>, wherein gas bubble segmentation is not carried out after the gas-liquid separation step.
<6> The method as described in any one of <1> to <5>, wherein the oxidizer is added in an amount which is excessive relative to the mercury in the sample.
<7> The method as described in any one of <1> to <6>, further including a nitric acid-hydrochloric acid step of adding nitric acid and hydrochloric acid to the sample to which the oxidizer has been added.
<8> The method as described in any one of <1> to <7>, wherein in the analysis step, inductively coupled plasma mass spectrometry or inductively coupled plasma optical emission spectrometry is used to carry out the quantitative analysis or the qualitative analysis.

### Examples

The present invention will be described in more detail with reference to examples below. Note, however, that the present invention is not limited to the examples, and the present invention also encompasses, in its scope, any example derived by combining, as appropriate, technical means disclosed in differing examples.

### [Device]

As a flow analyzer, used was an analyzer that was the flow analyzer illustrated in Fig. 1, the part B, illustrated in Fig. 1, of which had a configuration illustrated in Fig. 4 and which did not include a reagent introduction section 3. The flow analyzer includes: a sample introduction section 1 that is for introducing a sample into a tube 2; a marker introduction section 8 that is for introducing a marker into the tube 2; a gas bubble segmentation section 10 that carries out gas bubble segmentation with respect to the sample and the marker which have been introduced into the tube 2; a heating section 5 that carries out a heat treatment with respect to the sample which is transferred through the tube 2; a cooling section 6 that cools the sample which has been subjected to the heat treatment; a gas-liquid separation section 7 that removes gas which is present in the tube after cooling; an analysis section 4 that quantitatively or qualitatively analyzes the sample from which the gas has been removed by the gas-liquid separation section 7; and a marker detection section 9 that detects the marker and outputs a detection signal to the analysis section 4. The analysis section 4 acquires analysis data on the basis of the detection signal. Between the gas-liquid separation section 7 and the analysis section 4, a gas bubble segmentation section that newly introduces gas into the tube is not provided.

Air was used as gas for the gas bubble segmentation, and introduced into the tube 2 every 4 seconds. As the marker, palladium was used. The palladium was introduced into the tube 2 as a nitric acid acidic solution of palladium (the concentration of palladium: 100 mg/L). The nitric acid acidic solution of the palladium and the sample were alternately introduced into the tube 2. As a detection device of the marker detection section 9, a spectrophotometer (SCIC4000, manufactured by BL TEC K. K.) was used. As an analyzer of the analysis section 4, an inductively coupled plasma mass spectrometer (Agilent 7800 ICP-MS, manufactured by Agilent Technologies Japan, Ltd.) was used.

A pressure of 0.12 MPa, which applied pressure against the flow of the sample, was applied to the heating section 5 from downstream of the heating section 5 by a compressor. A heating temperature in the heating section 5 was set such that the sample had a temperature of 100°C.

A PFA tube having a length of 1.5 m and an inner diameter of 2.0 mm was used for the cooling section 6. The tube was constituted by a linear part having a length of 1.2 m, a coil part having a length of 0.6 m, and a linear part having a length of 0.3 m, in order from the outlet of the heating section 5. The number of turns of the coil part was 5 turns. The coil diameter of the coil part was 40 mm. The coil pitch of the coil part was 3.0 mm. Cooling was carried out by air cooling. The ambient temperature in the cooling section 6 was 15°C.

The marker detection section 9 and the analysis section 4 are disposed in series as illustrated in Fig. 4. The sample which has been subjected to the removal by the gas-liquid separation section 7 is continuously measured by the marker detection section 9 at a stage prior to introduction into the analysis section 4, and then transferred to the analysis section 4. The marker detection section 9 outputted the detection signal to the analysis section 4 upon detection of the marker, and the analysis section 4 started acquiring the analysis data upon receipt of the detection signal.

### [Example 1]

### <1. Preparation of sample solution, internal standard solution, and wash water>

The flow analyzer was used to measure the concentrations of metallic elements in a sample. As the sample, environmental water, waste water, or treated water listed in Table 1 below was used.

Nitric acid for electronics industry (70%), hydrochloric acid for electronics industry (35%), and sodium hypochlorite with 10% effective chlorine were added to each sample to prepare a sample solution. The volumes of the nitric acid, the hydrochloric acid, and the sodium hypochlorite relative to the volume of the sample were set to 1%, 0.2%, and 0.02%, respectively. Ultrapure water was used instead of each sample to measure an oxidation-reduction potential of a blank solution that had been similarly prepared. A measured value was +1.38 V. An oxidation-reduction potential of the sample solution is considered to be independent of each sample, and thus can be said to be +1.38 V. Note that the oxidation-reduction potential was measured with use of an oxidation-reduction potential meter (ORP meter).

Nitric acid for electronics industry (70%), hydrochloric acid for electronics industry (35%), and sodium hypochlorite with 10% effective chlorine were added also to a mercury standard solution to prepare an internal standard solution. The volumes of the nitric acid, the hydrochloric acid, and the sodium hypochlorite relative to the volume of the mercury standard solution were set to be the same as those in the sample solution. Similarly to the above, an oxidation-reduction potential of the internal standard solution can be said to also be +1.38 V.

Nitric acid for electronics industry (70%) and hydrochloric acid for electronics industry (35%) were added to pure water to prepare sampler wash water serving as a base. The volumes of the nitric acid and the hydrochloric acid relative to the volume of the pure water were set to be the same as those in the sample solution.

### <2. Sample analysis 1 (with addition of sodium hypochlorite)>

The internal standard solution was added to each sample solution so as to achieve 0.5 µg/L, which is an environmental standard value of mercury, and addition and recovery tests were carried out. After the addition and recovery tests were finished for each sample, the sampler wash water was circulated through the flow analyzer to wash a device flow path.

### <3. Sample analysis 2 (without addition of sodium hypochlorite)>

A sample solution and an internal standard solution were prepared by a method similar to that in 1. above, except that sodium hypochlorite with 10% effective chlorine was not added. The sample solution and the internal standard solution were used to carry out addition and recovery tests by a method similar to that in 2. above. Note that, in the analysis 2, ultrapure water was used instead of each sample to measure an oxidation-reduction potential of a blank solution that had been similarly prepared. A measured value was +0.66 V. An oxidation-reduction potential of the sample solution is considered to be independent of each sample, and thus can be said to be +0.66 V.

Test results obtained in the above analysis 1 and test results obtained in the above analysis 2 were compared. Table 1 shows comparison results.

**[Table 1]**

| | Sodium hypochlorite with addition of acid | | Sodium hypochlorite without addition of acid | |
|---|---|---|---|---|
| Sample | Measurement result (µg/L) | Recovery rate (%) | Measurement result (µg/L) | Recovery rate (%) |
| 10-fold diluted factory raw water | 0.4927 | 99% | 0.2593 | 52% |
| 10-fold diluted influent water | 0.4612 | 92% | 0.5170 | 103% |
| 10-fold diluted aerated water | 0.5064 | 101% | 0.3645 | 73% |
| Under 10-fold diluted seepage water | 0.5238 | 105% | 0.5015 | 100% |
| 10-fold diluted seawater upper layer | 0.5156 | 103% | 0.4380 | 88% |
| 10-fold diluted seawater upper layer | 0.5069 | 101% | 0.4166 | 83% |
| 10-fold diluted seawater middle layer | 0.5034 | 101% | 0.4082 | 82% |
| 10-fold diluted seawater lower layer | 0.5119 | 102% | 0.4358 | 87% |
| Under effluent | 0.5759 | 115% | 0.3340 | 67% |
| Under effluent | 0.5257 | 105% | 0.4254 | 85% |
| Under effluent | 0.4917 | 98% | 0.4831 | 97% |
| Under river water | 0.5157 | 103% | 0.4325 | 86% |
| Under river water | 0.5105 | 102% | 0.4239 | 85% |
| Under river water | 0.5252 | 105% | 0.4098 | 82% |
| Under river water | 0.5198 | 104% | 0.4120 | 82% |
| Lake water under reservoir upper layer | 0.5217 | 104% | 0.4390 | 88% |
| Lake water under reservoir upper layer | 0.5237 | 105% | 0.4259 | 85% |
| Lake water under reservoir upper layer | 0.5298 | 106% | 0.4529 | 91% |
| Lake water under reservoir lower layer | 0.5596 | 112% | 0.4396 | 88% |
| Under swamp water | 0.5005 | 100% | 0.4516 | 90% |
| 10-fold diluted sludge eluate | 0.4961 | 99% | 0.4237 | 85% |
| 10-fold diluted catalyst eluate | 0.5087 | 102% | 0.4348 | 87% |
| 50-fold diluted treated ash eluate | 0.541 | 108% | 0.1061 | 21% |
| 50-fold diluted treated ash eluate | 0.5267 | 105% | 0.2739 | 55% |
| 50-fold diluted treated ash eluate | 0.529 | 106% | 0.3715 | 74% |
| 50-fold diluted treated ash eluate | 0.5195 | 104% | 0.3861 | 77% |

In the table, a 10-fold diluted or 50-fold diluted sample was a sample having a large number of matrices or a sample having a high salt content, such as seawater. Thus, each reagent was added after dilution.

The results in Table 1 show that there was variation in recovery rate in a case where sodium hypochlorite was not added. In contrast, in a case where sodium hypochlorite was added, it was possible to obtain a good recovery rate.

### [Comparative Example 1]

The flow analyzer was used to measure the concentrations of metallic elements in a sample. As the sample, environmental water, waste water, or treated water listed in Table 2 below was used.

In Comparative Example 1, hydrogen peroxide considered to have high oxidizing power was used instead of sodium hypochlorite to carry out addition and recovery tests.

Specifically, nitric acid for electronics industry (70%), hydrochloric acid for electronics industry (35%), and hydrogen peroxide (approximately 30%) were added to each sample to prepare a sample solution. The volumes of the nitric acid, the hydrochloric acid, and the hydrogen peroxide relative to the volume of the sample were set to 1%, 0.2%, and 0.2%, respectively. Note that ultrapure water was used instead of each sample to measure an oxidation-reduction potential of a blank solution that had been similarly prepared. A measured value was +0.77 V. An oxidation-reduction potential of the sample solution is considered to be independent of each sample, and thus can be said to be +0.77 V.

Nitric acid for electronics industry (70%), hydrochloric acid for electronics industry (35%), and hydrogen peroxide (approximately 30%) were added also to a mercury standard solution to prepare an internal standard solution. The volumes of the nitric acid, the hydrochloric acid, and the hydrogen peroxide relative to the volume of the mercury standard solution were set to be the same as those in the sample solution. Similarly to the above, an oxidation-reduction potential of the internal standard solution can be said to also be +0.77 V.

Sampler wash water serving as a base was prepared as in the case of Example 1.

The internal standard solution was added to each sample solution so as to achieve 0.5 µg/L, which is an environmental standard value of mercury, and addition and recovery tests were carried out. After the addition and recovery tests were finished for each sample, the sampler wash water was circulated through the flow analyzer to wash a device flow path. Table 2 shows results of the addition and recovery tests.

**[Table 2]**

| Sample | Measurement result (µg/L) | Recovery rate (%) |
|---|---|---|
| 10-fold diluted factory raw water | 0.3055 | 61% |
| 10-fold diluted influent water | 0.4184 | 84% |
| 10-fold diluted aerated water | 0.3279 | 66% |
| Under seepage water | 0.4381 | 88% |
| 10-fold diluted seawater upper layer | 0.4018 | 80% |
| 10-fold diluted seawater upper layer | 0.3978 | 80% |
| 10-fold diluted seawater middle layer | 0.3850 | 77% |
| 10-fold diluted seawater lower layer | 0.4247 | 85% |
| Under effluent | 0.3423 | 68% |
| Under effluent | 0.3389 | 68% |
| Under effluent | 0.4362 | 87% |
| Under effluent | 0.3678 | 74% |
| Under river water | 0.3429 | 69% |
| Under river water | 0.3774 | 75% |
| Under river water | 0.3565 | 71% |
| Under river water | 0.3376 | 68% |
| Lake water under reservoir upper layer | 0.3379 | 68% |

The results in Table 2 show that, in a case where hydrogen peroxide was used as an oxidizer, it was impossible to obtain a good recovery rate.

### [Example 2]

In Example 2, the concentrations of metal elements in a sample were manually measured without use of the flow analyzer. As the sample, environmental water, waste water, or treated water listed in Table 3 below was used.

In a beaker, 50 mL of the sample was placed. Nitric acid for electronics industry (70%), hydrochloric acid for electronics industry (35%), and sodium hypochlorite with 10% effective chlorine were added to the sample to prepare a sample solution. The volumes of the nitric acid, the hydrochloric acid, and the sodium hypochlorite relative to the volume of the sample were set to 1%, 0.2%, and 0.02%, respectively. A mercury standard solution was added to the sample solution so as to achieve 0.5 µg/L, which is an environmental standard value of mercury. A hot plate was set to 100°C to thermally decompose, for 3 hours, the sample solution thus adjusted.

The sample solution was allowed to cool and then diluted in a measuring flask to 50 mL with ultrapure water, and the mercury concentration was measured by ICP-MS. Table 3 shows measurement results. Note that ultrapure water was used instead of each sample to measure an oxidation-reduction potential of a blank solution that had been similarly prepared. A measured value was +1.38 V. An oxidation-reduction potential of the sample solution is considered to be independent of each sample, and thus can be said to be +1.38 V.

**[Table 3]**

| Sample | Measurement result (µg/L) | Recovery rate (%) |
|---|---|---|
| Treatment solution obtained by 10-fold dilution of raw water at liquid embedding facility | 0.4131 | 83% |
| 10-fold diluted influent water | 0.5094 | 102% |
| Effluent | 0.5165 | 103% |
| 10-fold diluted seawater upper layer | 0.5125 | 103% |
| 10-fold diluted seawater middle layer | 0.5218 | 104% |
| 10-fold diluted seawater lower layer | 0.5104 | 102% |
| Treatment plant surplus water 1 | 0.5070 | 101% |
| Treatment plant surplus water 2 | 0.5359 | 107% |
| Treatment facility treated water | 0.5126 | 103% |
| 10-fold diluted aeration tank aerated water | 0.5262 | 105% |
| Nitrogen plant treated water | 0.4981 | 100% |
| Lake water reservoir upper layer | 0.4841 | 97% |
| Lake water reservoir upper layer | 0.4875 | 98% |
| Lake water reservoir upper layer | 0.4810 | 96% |

The results in Table 3 show that, even in a case where the flow analyzer was not used, using sodium hypochlorite as an oxidizer made it possible to obtain a good recovery rate.

### [Comparative Example 2]

The mercury concentration in a sample was measured by a method similar to that in Example 2, except that a sample listed in Table 4 below was used and that sodium hypochlorite with 10% effective chlorine was not added. Table 4 shows measurement results. Note that ultrapure water was used instead of each sample to measure an oxidation-reduction potential of a blank solution that had been similarly prepared. A measured value was +0.66 V. An oxidation-reduction potential of the sample solution is considered to be independent of each sample, and thus can be said to be +0.66 V.

**[Table 4]**

| Sample | Measurement result (µg/L) | Recovery rate (%) |
|---|---|---|
| 10-fold diluted factory raw water | 0.3119 | 62% |
| 10-fold diluted influent water | 0.459 | 92% |
| 10-fold diluted aerated water | 0.3152 | 63% |
| Under seepage water | 0.4795 | 96% |
| 10-fold diluted seawater upper layer | 0.4466 | 89% |
| 10-fold diluted seawater upper layer | 0.4393 | 88% |
| 10-fold diluted seawater middle layer | 0.4211 | 84% |
| 10-fold diluted seawater lower layer | 0.4294 | 86% |
| Effluent | 0.4169 | 83% |
| Effluent | 0.3970 | 79% |
| Effluent | 0.4820 | 96% |
| Effluent | 0.4178 | 84% |
| River water | 0.4576 | 92% |
| River water | 0.4589 | 92% |
| River water | 0.4437 | 89% |
| River water | 0.4720 | 94% |
| Lake water reservoir upper layer | 0.4754 | 95% |

The results in Table 4 show that, as compared with the results of Example 2 (Table 3), it was impossible to obtain a good recovery rate.

### [Example 3]

In Example 3, the flow analyzer was used to measure the concentrations of metal elements in a sample. As the sample, environmental water, waste water, or treated water listed in Table 5 below was used.

A sample solution, an internal standard solution, and wash water were prepared by a method similar to that in <1. Preparation of sample solution, internal standard solution, and wash wa-ter> of Example 1, except that hydrochloric acid for electronics industry (35%) was not added to each sample.

The internal standard solution was added to each sample solution so as to achieve 0.5 µg/L, which is an environmental standard value of mercury, and addition and recovery tests were carried out. After the addition and recovery tests were finished for each sample, the sampler wash water was circulated through the flow analyzer to wash a device flow path. Table 5 shows results.

**[Table 5]**

| Sample | Measurement result (µg/L) | Recovery rate (%) |
|---|---|---|
| 10-fold diluted factory raw water | 0.4756 | 95% |
| 10-fold diluted influent water | 0.4960 | 99% |
| 10-fold diluted aerated water | 0.4893 | 98% |
| Under seepage water | 0.5114 | 102% |
| 10-fold diluted seawater upper layer | 0.4473 | 89% |
| 10-fold diluted seawater upper layer | 0.4270 | 85% |
| 10-fold diluted seawater middle layer | 0.4137 | 83% |
| 10-fold diluted seawater lower layer | 0.4143 | 83% |
| Under effluent | 0.4778 | 96% |
| Under effluent | 0.4900 | 98% |

The results in Table 5 show that using sodium hypochlorite as an oxidizer made it possible to obtain a good recovery rate, even without addition of hydrochloric acid.

### [Comparative Example 3]

Mercury standard solutions each containing mercury in a concentration range of 0.025 µg/L to 1 µ/L were prepared. To the prepared mercury standard solutions having respective concentrations, nitric acid for electronics industry (70%) and hydrochloric acid for electronics industry (35%) were added to prepare internal standard solutions. The flow analyzer was used to measure mercury concentrations of the internal standard solutions having the respective mercury concentrations, so that a calibration curve was created. Note that the volumes of the nitric acid and the hydrochloric acid relative to the volume of each of the mercury standard solutions were set to 1% and 0.2%, respectively. Note that ultrapure water was used instead of each sample to measure an oxidation-reduction potential of a blank solution that had been similarly prepared. A measured value was +0.66 V. An oxidation-reduction potential of the sample solution is considered to be independent of each sample, and thus can be said to be +0.66 V.

Nitric acid for electronics industry (70%) and hydrochloric acid for electronics industry (35%) were added to pure water to also prepare sampler wash water serving as a base. The volumes of the nitric acid and the hydrochloric acid relative to the volume of the pure water were set to 1% and 0.2%, respectively.

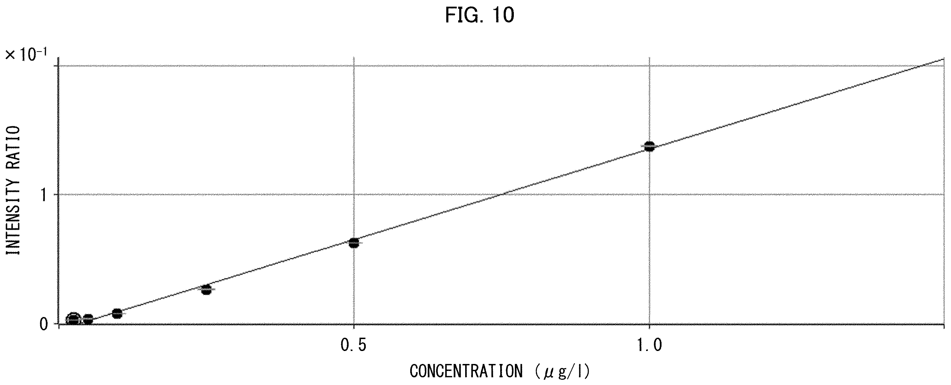

A resulting calibration curve had a coefficient of determination R of 0.9983 as illustrated in Fig. 10. Further, it can be seen that the calibration curve is slightly curved.

### [Example 4]

An internal standard solution and sampler wash water were prepared by a method similar to that in Comparative Example 3, except that sodium hypochlorite with 10% effective chlorine was added. The volumes of the nitric acid, the hydrochloric acid, and the sodium hypochlorite relative to the volume of the mercury standard solution were set to 1%, 0.2%, and 0.02%, respectively, in each of the internal standard solutions. Further, the volumes of the nitric acid, the hydrochloric acid, and the sodium hypochlorite relative to the volume of the pure water were set to 1%, 0.2%, and 0.02%, respectively, in the sampler wash water. Note that ultrapure water was used instead of each sample to measure an oxidation-reduction potential of a blank solution that had been similarly prepared. A measured value was +1.38 V. An oxidation-reduction potential of the sample solution is considered to be independent of each sample, and thus can be said to be +1.38 V.

The flow analyzer was used to measure mercury concentrations of the internal standard solutions having the respective mercury concentrations, so that a calibration curve was created.

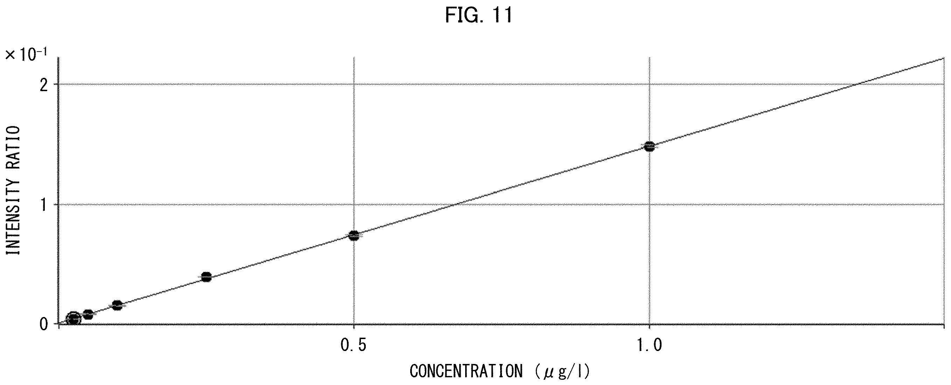

A resulting calibration curve had a coefficient of determination R of 0.9999 as illustrated in Fig. 11, so that it was possible to obtain good linearity.

Then, recovery rates of measured concentration values of mercury standard solutions that had respective concentrations which had been quantified with use of the calibration curve illustrated in Fig. 10 or Fig. 11 were calculated. Table 6 shows results.

**[Table 6]**

| | Sodium hypochlorite with addition of acid | | Sodium hypochlorite without addition of acid | |
|---|---|---|---|---|
| Hg concentration (µg/L) | Measurement result (µg/L) | Recovery rate (%) | Measurement result (µg/L) | Recovery rate (%) |
| 0.025 | 0.023 | 92% | 0.053 | 214% |
| 0.05 | 0.049 | 98% | 0.062 | 124% |
| 0.1 | 0.099 | 99% | 0.090 | 90% |
| 0.25 | 0.260 | 104% | 0.223 | 89% |
| 0.5 | 0.494 | 99% | 0.481 | 96% |
| 1 | 1.001 | 100% | 1.016 | 102% |

The results in Table 6 show that a recovery rate at or near a low concentration was markedly low in a case where sodium hypochlorite was not added to a mercury standard solution. In contrast, in a case where sodium hypochlorite was added to a standard solution, it was possible to obtain a good recovery rate in all concentration ranges.

### [Example 4]

The flow analyzer was used to measure the concentrations of metallic elements in a sample. As the sample, environmental water, waste water, or treated water listed in Table 7 below was used.

**In** Example 4, potassium permanganate considered to have high oxidizing power was used instead of sodium hypochlorite to carry out addition and recovery tests.

Specifically, nitric acid for electronics industry (70%), hydrochloric acid for electronics industry (35%), and potassium permanganate were added to each sample to prepare a sample solution. The volumes of the nitric acid and the hydrochloric acid relative to the volume of the sample were set to 1% and 0.2%, respectively. Further, potassium permanganate was added in an amount of 20 mg/L relative to the volume of the sample. Note that ultrapure water was used instead of each sample to measure an oxidation-reduction potential of a blank solution that had been similarly prepared. A measured value was +1.24 V. An oxidation-reduction potential of the sample solution is considered to be independent of each sample, and thus can be said to be +1.24 V.

Nitric acid for electronics industry (70%), hydrochloric acid for electronics industry (35%), and potassium permanganate were added also to a mercury standard solution to prepare an internal standard solution. The volumes of the nitric acid and the hydrochloric acid relative to the volume of the mercury standard solution were set to be the same as those in the sample solution. The amount of the potassium permanganate relative to the volume of the mercury standard solution was also set to be the same as that in the sample solution. Similarly to the above, an oxidation-reduction potential of the internal standard solution can be said to also be +1.24 V.

Sampler wash water serving as a base was prepared as in the case of Example 1.

The internal standard solution was added to each sample solution so as to achieve 0.5 µg/L, which is an environmental standard value of mercury, and addition and recovery tests were carried out. After the addition and recovery tests were finished for each sample, the sampler wash water was circulated through the flow analyzer to wash a device flow path. Table 7 shows results of the addition and recovery tests.

**[Table 7]**

| Sample | Measurement result (µg/L) | Recovery rate (%) |
|---|---|---|
| 10-fold diluted factory raw water | 0.4954 | 99% |
| 10-fold diluted influent water | 0.487 | 97% |
| 10-fold diluted aerated water | 0.4918 | 98% |
| Seepage water | 0.4861 | 97% |
| 10-fold diluted seawater upper layer | 0.4688 | 94% |
| 10-fold diluted seawater upper layer | 0.4494 | 90% |
| 10-fold diluted seawater middle layer | 0.4428 | 89% |
| 10-fold diluted seawater lower layer | 0.4351 | 87% |
| 10-fold diluted effluent | 0.4591 | 92% |
| Effluent | 0.4802 | 96% |

The results in Table 7 show that using potassium permanganate instead of sodium hypochlorite as an oxidizer also made it possible to obtain a good recovery rate.

### [Example 5]

Mercury has an oxidation-reduction potential of 0.7986 and is a metal that is easily reduced. In a case where Hg²⁺ in a sample solution is reduced to Hg, mercury easily vaporizes. In order to prevent mercury from being reduced, the sample solution is desirably in an oxidation state. Therefore, oxidation-reduction potentials of sample solutions each containing potassium permanganate, sodium hypochlorite, or hydrogen peroxide used in Examples 1 to 4 or Comparative Examples 1 to 3 were measured.

Specifically, oxidation-reduction potentials of the following blank solutions A to J were measured.
A: a solution obtained by adding nitric acid for electronics industry (70%) to pure water so that the volume of the nitric acid would be 1%;
B: a solution obtained by adding nitric acid for electronics industry (70%) and hydrochloric acid for electronics industry (35%) to pure water so that the volumes of the nitric acid and the hydrochloric acid would be 1% and 0.2%, respectively;
C: a solution obtained by adding sodium hypochlorite with 10% effective chlorine to the solution B so that the volume of the sodium hypochlorite would be 0.002%;
D: a solution obtained by adding sodium hypochlorite with 10% effective chlorine to the solution B so that the volume of the sodium hypochlorite would be 0.02%;
E: a solution obtained by adding sodium hypochlorite with 10% effective chlorine to the solution B so that the volume of the sodium hypochlorite would be 0.2%;
F: a solution obtained by adding hydrogen peroxide to the solution B so that the volume of the hydrogen peroxide would be 0.02%;
G: a solution obtained by adding hydrogen peroxide to the solution B so that the volume of the hydrogen peroxide would be 0.2%;
H: a solution obtained by adding potassium permanganate to the solution B so that the amount of the potassium permanganate added would be 2 mg/L;
I: a solution obtained by adding potassium permanganate to the solution B so that the amount of the potassium permanganate added would be 20 mg/L; and
J: a solution obtained by adding potassium permanganate to the solution B so that the amount of the potassium permanganate added would be 200 mg/L.

Table 8 shows results.

**[Table 8]**

| | Sample | Oxidation-reduction potential (V) |
|---|---|---|
| A | 1 ml of nitric acid/100 mL | +0.66 |
| B | (1 ml of nitric acid + 0.2 ml of hydrochloric acid)/100 ml | +0.66 |
| C | B + 0.002% sodium hypochlorite | +0.86 |
| D | B + 0.02% sodium hypochlorite | +1.38 |
| E | B + 0.2% sodium hypochlorite | +1.42 |
| F | B + 0.02% hydrogen peroxide | +0.77 |
| G | B + 0.2% hydrogen peroxide | +0.77 |
| H | B + 2 mg/L of potassium permanganate | +1.13 |
| I | B + 20 mg/L of potassium permanganate | +1.24 |
| J | B + 200 mg/L of potassium permanganate | +1.31 |

It has been found from Table 8 that the blank solutions C to E each containing sodium hypochlorite and the blank solutions H to J each containing potassium permanganate had high oxidation-reduction potentials. It has been inferred that it was possible to obtain a good recovery rate because of the following reason: Since these blank solutions make it possible to oxidize mercury and keep the mercury in a state of Hg²⁺, it is possible to carry out measurement without vaporization of mercury by reduction.

Note that an oxidation-reduction potential of a sample solution prepared with a composition similar to that of a blank solution with respect to a sample is considered to be substantially independent of an oxidation-reduction potential of a target substance in the sample which target substance is contained in a very small amount. This makes it possible to say that the oxidation-reduction potential of the sample solution is the same as that of the blank solution. It is therefore possible to say that oxidation-reduction potentials of sample solutions prepared with the same compositions as those of the blank solutions A to J are the oxidation-reduction potentials of the blank solutions A to J. Note that the sample solutions which were used in Examples 1 to 4 and which have higher oxidation-reduction potentials than mercury are based on the compositions of the blank solutions D and I.

### [Example 6]

In Example 6, the flow analyzer was used to simultaneously measure the concentrations of metallic elements in a sample. As the sample, mineral water to which various metals standard solutions of metals listed in Table 9 had been added at given concentrations was used.

Nitric acid for electronics industry (70%), hydrochloric acid for electronics industry (35%), sodium hypochlorite with 10% effective chlorine, and a metal standard solution listed in Table 9 were added to the mineral water to prepare a sample solution. The volumes of the nitric acid, the hydrochloric acid, and the sodium hypochlorite relative to the volume of the mineral water were set to 1%, 0.2%, and 0.02%, respectively. Sampler wash water serving as a base was prepared as in the case of Example 1. Ultrapure water was used instead of mineral water to measure an oxidation-reduction potential of a blank solution that had been similarly prepared. A measured value was +1.38 V. An oxidation-reduction potential of the sample solution is considered to be independent of mineral water, and thus can be said to be +1.38 V.

The flow analyzer was used to carry out a recovery test with respect to the sample solution. Note that a value obtained by subtracting the concentration of a metal which had been contained in the mineral water from obtained measured concentration values of various metals was regarded as the metal concentration of the sample solution. Table 9 shows results.

**[Table 9]**

| Item | B | | Al | | Cr | | Mn | | | |
|---|---|---|---|---|---|---|---|---|---|---|
| Set value | 1/10 of reference | Reference value (µg/L) | 1/10 of reference value (µg/L) | Reference value (µg/L) | 1/10 of reference value (µg/L) | Reference value (µg/L) | 1/10 of reference value (µg/L) | | Reference value (µg/L) 50 | |
| | value (µg/L) 100 | 1000 | 20 | 200 | 2 | 20 | 5 | | | |
| First time | 94.323 | 969.486 | 19.152 | 196.366 | 1.962 | 20.328 | 4.858 | | 48.429 | |
| Second time | 100.236 | 968.249 | 20.261 | 193.569 | 2.192 | 19.911 | 5.14 | | 48.499 | |
| Third time | 96.36 | 967.103 | 19.680 | 199.334 | 2.011 | 19.570 | 4.578 | | 48.479 | |
| Fourth time | 98.007 | 994.617 | 19.676 | 193.941 | 1.956 | 19.741 | 4.896 | | 48.706 | |
| Fifth time | 96.532 | 997.888 | 19.609 | 203.499 | 1.895 | 20.069 | 4.861 | | 48.916 | |
| Average value | 97.092 | 979.469 | 19.676 | 197.342 | 2.003 | 19.924 | 4.867 | | 48.606 | |
| Trueness (%) | 97.1 | 97.9 | 98.4 | 98.7 | 100.2 | 99.6 | 97.3 | | 97.2 | |
| Standard deviation | 2.193 | 15.388 | 0.394 | 4.142 | 0.113 | 0.293 | 0.200 | | 0.203 | |
| RSD (%) | 2.3 | 1.6 | 2.000 | 2.1 | 5.7 | 1.5 | 4.1 | | 0.4 | |

| Item | Fe | | Cu | | | | Zn | | | |
|---|---|---|---|---|---|---|---|---|---|---|
| Set value | 1/10 of reference value (µg/L) | Reference value (µg/L) | 1/10 of reference value (µg/L) | | Reference value (µg/L) | | 1/10 of reference value (µg/L) | | Reference value (µg/L) | |
| | 30 | 300 | 100 | | 1000 | | 100 | | 1000 | |
| First time | 28.578 | 297.155 | 98.988 | | 973.471 | | 94.803 | | 1000.667 | |
| Second time | 31.418 | 294.167 | 102.769 | | 951.035 | | 91.382 | | 974.301 | |
| Third time | 27.242 | 295.447 | 96.911 | | 963.945 | | 93.886 | | 979.989 | |
| Fourth time | 29.946 | 297.291 | 97.714 | | 960.389 | | 95.047 | | 1002.259 | |
| Fifth time | 27.990 | 294.820 | 96.398 | | 952.967 | | 81.233 | | 967.921 | |
| Average value | 29.035 | 295.776 | 98.556 | | 960.361 | | 91.270 | | 985.027 | |
| Trueness (%) | 96.8 | 98.6 | 98.6 | | 96.0 | | 91.3 | | 98.5 | |
| Standard deviation | 1.660 | 1.397 | 2.550 | | 9.033 | | 5.795 | | 15.609 | |
| RSD (%) | 5.7 | 0.5 | 2.6 | | 0.9 | | 6.3 | | 1.6 | |

| Item | As | | Se | | Cd | | Pb | | Hq | |
|---|---|---|---|---|---|---|---|---|---|---|
| Set value | 1/10 of reference value (µg/L) | Reference value (µg/L) | 1/10 of reference value (µg/L) | Reference value (µg/L) | 1/10 of reference value (µg/L) | Reference value (µg/L) | 1/10 of reference value (µg/L) | Reference value (µg/L) | 1/10 of reference value (µg/L) | Reference value (µg/L) |
| | 1 | 10 | 1 | 10 | 0.3 | 3 | 1 | 10 | 0.05 | 0.5 |
| First time | 1.013 | 10.477 | 0.996 | 10.100 | 0.284 | 2.926 | 0.932 | 9.782 | 0.052 | 0.522 |
| Second time | 1.134 | 10.107 | 1.089 | 9.801 | 0.310 | 2.957 | 1.072 | 9.769 | 0.054 | 0.499 |
| Third time | 1.017 | 9.938 | 0.975 | 9.476 | 0.297 | 2.854 | 0.998 | 9.421 | 0.050 | 0.490 |
| Fourth time | 0.990 | 10.084 | 0.991 | 9.936 | 0.303 | 3.061 | 1.019 | 9.960 | 0.052 | 0.515 |
| Fifth time | 0.997 | 10.231 | 0.967 | 9.905 | 0.285 | 2.976 | 0.968 | 10.039 | 0.048 | 0.515 |
| Average value | 1.030 | 10.167 | 1.004 | 9.843 | 0.296 | 2.955 | 0.998 | 9.794 | 0.051 | 0.508 |
| Trueness (%) | 103.0 | 101.7 | 100.4 | 98.4 | 98.5 | 98.5 | 99.8 | 97.9 | 102.2 | 101.7 |
| Standard deviation | 0.059 | 0.202 | 0.049 | 0.232 | 0.011 | 0.075 | 0.053 | 0.238 | 0.002 | 0.013 |
| RSD (%) | 5.7 | 2.0 | 4.9 | 2.4 | 3.8 | 2.6 | 5.3 | 2.4 | 4.9 | 2.6 |

As shown in Table 9, it was possible to obtain good recovery rates in all metal items. This established a method for using a flow analysis method to measure metals including mercury.

### Industrial Applicability

According to the present invention, it is possible to provide an analysis method and a flow analysis method each of which makes it possible to continuously analyze, with high accuracy, a sample containing mercury.

According to such a configuration, it is possible to contribute to reduction of pollution of environmental water, tap water, and the like. Thus, it is possible to contribute to achievement of Goals 14 and 15 of the Sustainable Development Goals (SDGs).

### Reference Signs List

- 1: Sample introduction section
- 2: Tube
- 3: Reagent introduction section
- 4: Analysis section
- 5: Heating section
- 6: Cooling section
- 7: Gas-liquid separation section
- 8: Marker introduction section
- 9: Marker detection section
- 10: Gas bubble segmentation section
- 11: Carrier introduction section
- 12: Pressurization section
- 13: Gas bubbles
- 14: Gas bubbles

## Claims

1. A method for analyzing a sample containing mercury, comprising:
- a pretreatment step of adding an oxidizer to a sample containing mercury to prepare a sample solution so as to achieve an oxidation state in which an oxidation-reduction potential is not less than +0.80 V; and
- an analysis step of subjecting the sample solution which has been prepared in the pretreatment step to a quantitative analysis or a qualitative analysis while maintaining the oxidation state.

2. A method for analyzing a sample containing mercury,
- the method being carried out by a flow analysis method including:
- a sample introduction step of introducing, into a tube, a sample containing mercury; and
- an analysis step of carrying out a quantitative analysis or a qualitative analysis with respect to the sample, said method comprising:
- a pretreatment step of, prior to the sample introduction step, adding an oxidizer to the sample containing mercury to prepare a sample solution so as to achieve an oxidation-reduction potential of not less than +0.80 V and/or a reagent addition step of adding the oxidizer to the sample so as to achieve an oxidation-reduction potential of not less than +0.80 V, the sample being transferred inside the tube;
- a heating step of carrying out a heat treatment with respect to the sample to which the oxidizer has been added;
- a cooling step of cooling the sample which has been subjected to the heat treatment and which is transferred inside the tube; and
- a gas-liquid separation step of removing gas which is present in the tube after cooling,
- a quantitative analysis or a qualitative analysis being carried out with respect to the sample from which the gas has been removed in the gas-liquid separation step.

3. The method as set forth in claim 1 or 2, wherein the oxidizer is at least any one selected from the group consisting of hypochlorous acid or a salt thereof and permanganic acid or a salt thereof.

4. The method as set forth in claim 2, further comprising a gas bubble segmentation step of producing, inside the tube, a plurality of segments separated by gas bubbles, by carrying out gas bubble segmentation with respect to the sample which is introduced into the tube.

5. The method as set forth in claim 2 or 4, wherein gas bubble segmentation is not carried out after the gas-liquid separation step.

6. The method as set forth in claim 1 or 2, wherein the oxidizer is added in an amount which is excessive relative to the mercury in the sample.

7. The method as set forth in claim 1 or 2, further comprising a nitric acid-hydrochloric acid step of adding nitric acid and hydrochloric acid to the sample to which the oxidizer has been added.

8. The method as set forth in claim 1 or 2, wherein in the analysis step, inductively coupled plasma mass spectrometry or inductively coupled plasma optical emission spectrometry is used to carry out the quantitative analysis or the qualitative analysis.
